# EUROPEAN PATENT SPECIFICATION

(11) **EP 3 883 522 B1**
(45) Date of publication and mention of the grant of the patent: **08.01.2025**
(21) Application number: 19805408.2
(22) Date of filing: 11.11.2019
(51) Int. Cl.: A61K 6/90, A61K 6/891, A61K 6/00, A61K 6/18

(54) **USE OF A POLYETHER-MODIFIED POLYDIMETHYL SILOXANE FOR REDUCING THE STICKINESS OF A DENTAL IMPRESSION COMPOSITION**
VERWENDUNG EINES POLYETHERMODIFIZIERTEN POLYDIMETHYLSILOXANS ZUR REDUZIERUNG DER KLEBRIGKEIT EINES DENTALEN ABFORMMATERIALS
UTILISATION D'UN POLYDIMÉTHYL SILOXANE MODIFIÉ AU POLYÉTHER POUR RÉDUIRE L'ADHÉRANCE D'UN MATÉRIAU D'EMPREINTE DENTAIRE

(30) Priority: 20.11.2018 EP 18207289
(43) Date of publication of application: 29.09.2021
(73) Proprietor: Solventum Intellectual Properties Company, Maplewood, MN 55144 (US)
(72) Inventor: ZECH, Joachim W., 82229 Seefeld (DE); KUPPERMANN, Bernd, 82229 Seefeld (DE); GRUPP, Hendrik, 82229 Seefeld (DE)
(74) Representative: Brem, Roland
(86) International application number: PCT/IB2019/059676
(87) International publication number: WO 2020/104889

(56) References cited:
- WO-A1-2012/170413
- US-A1- 2008 200 585
- US-A1- 2008 319 100
- US-A1- 2017 233 531
- DATABASE REGISTRY [online] CHEMICAL ABSTRACTS SERVICE, COLUMBUS, OHIO, US; 16 November 1984 (1984-11-16), "Siloxane and silicone, di-Me, 3-hydroxypropyl Me, ethers with polyethylene glycol mono-Me ether", XP002796739, Database accession no. 68938-54-5
- DATABASE REGISTRY [online] CHEMICAL ABSTRACTS SERVICE, COLUMBUS, OHIO, US; 16 November 1984 (1984-11-16), "Siloxane and silicone, di-Me, 3-hydroxypropyl Me, ethers with polyethylene glycol mono-Me ether", XP002796739, Database accession no. 68938-54-5
- ANONYMOUS: "CAS#:68938-54-5 | Poly[dimethylsiloxane-co-methyl(3-hydroxypropyl)siloxane]-graft-poly(ethylene glycol) methyl ether | Chemsrc", 23 June 2019 (2019-06-23), XP055655927, Retrieved from the Internet <URL:https://www.chemsrc.com/en/cas/68938-54-5_1465704.html> [retrieved on 20200108]
- ANONYMOUS: "Poly[dimethylsiloxane-co-methyl(3-hydroxypropyl)siloxane]-graft-poly(ethylene glycol) methyl ether viscosity ~45 cSt (lit.) | Sigma-Aldrich", 8 January 2020 (2020-01-08), XP055656169, Retrieved from the Internet <URL:https://www.sigmaaldrich.com/catalog/product/aldrich/482412?lang=en ion=NL> [retrieved on 20200109]

## Description

### Field of the Invention

The invention relates to the use of a polyether-modified polydimethyl siloxane for reducing the stickiness of a dental composition after hardening, wherein the dental composition is useful for taking a dental impression and/or conducting a dental retraction step. The composition comprises a cationically curable pre-polymer with a polyether backbone and at least two reactive functional moieties and a suitable initiator for starting the curing reaction. The composition is provided as a kit of parts, wherein the parts are to be mixed shortly before use.

### Background

Dental impression materials are used to record the oral situation of a patient. The resulting hardened impression material captures the negative of the oral situation.

Most dental impression materials are typically provided as a kit of parts containing a base paste and a catalyst paste, which are mixed prior to their application. The mixed pastes are typically applied with the help of a dental tray and/or a syringe-type device. Usually the hardened material can be removed after about one to about six minutes after application.

The hardened impression material is used either for making a provisional restoration using a temporary crown and bridge material or for producing a positive model of the oral situation by casting the mould with e.g. gypsum. The obtained positive model is used for making the final restoration in the dental laboratory.
Different types of chemistry can be employed to formulate impression materials.

Often used are so-called polyether impression materials which cure by a cationic ring-opening polymerization of aziridines (e.g. Impregum^{™} 3M Oral Care). Aziridine moieties containing components are typically cured by using acids.
Even, if already hydrophilic by nature, sometimes further surfactants are added to these materials to make the composition even more hydrophilic and useful for taking dental impressions.

US 2004/0149164 A1 (Eckhardt et al.) describes a mixture of elongated N-alkylaziridine prepolymers which can be used as a dental impression material. The mixture can contain various modifiers like finely divided fillers, pigments, thixotropic agents and surface-active substances.

In US 5,569 691 (Guggenberger et al.) relates to a rubber-elastic composition comprising a polyether material which contains 0.1 to 15 wt.% of at least one hydrophilic nature imparting agent selected from the group of certain hydrophilic silicone oils, fluorinated hydrocarbons, EO/PO block copolymers, fatty alcohol derivatives, ethoxylated alkyl phenol, ethoxylated fatty amines and amine oxides.

In US 2008/200585 A1 (Klettke et al.) describes an initiator of a certain formula which is particular useful for initiating the curing reaction of cationic polymerizable compositions.

US 2008/0319100 A1 (Bublewitz et al.) describes a dental impression mass which contain selected curable polymer systems and a mixture of a silicone-containing non-ionic surfactant containing at least one partially fluorinated or perfluorinated hydrocarbon radical. One of the silicone-containing non-ionic surfactants used is PEG-8-methicone which is said to have a surface tension of 20.7 dyn/cm.

### Summary of Invention

As mentioned above, for certain application like dental impression materials an even more hydrophilic polyether material is desired, in particular as regards the so-called initial hydrophilicity, i.e. the hydrophilicity of the composition at the time when it is brought in contact with water or a wet surface.

The hydrophilicity of these materials can be improved e.g. by addition of certain polyether-based surfactants.

However, it was found that polyether-based dental impression materials sometimes show a kind of stickiness after hardening or tend to become sticky if stored in water, in particular if polyether surfactants are present.

From a practical point of view, this stickiness is not desirable as the practitioner might assume that the hardening process of the composition is not completed yet. Further, handling a hardened composition which is slightly sticky is not desired, either.

Thus, there is a need for a polyether-based composition, which can be used as dental material, and which shows a reduced stickiness after hardening.

If possible, other properties of the hardened composition such as tensile strength or elongation at break should not be negatively affected. One of more of the above objects are solved by the invention described in the present text

The invention relates to the use of a polyether-modified polydimethyl siloxane as described in the claims for reducing the stickiness of a dental impression composition provided as a kit of parts comprising a Base Paste and an Initiator Paste being separated before use, the Base Paste comprising a cationically curable pre-polymer comprising a polyether backbone and at least two reactive functional moieties, filler, the Initiator Paste comprising initiator being able to start the hardening of the polyether pre-polymer, filler, diluent, the Base Paste or the Catalyst Paste or the Base Paste and the Catalyst Paste comprising in addition a polyether-modified polydimethyl siloxane of the following formula 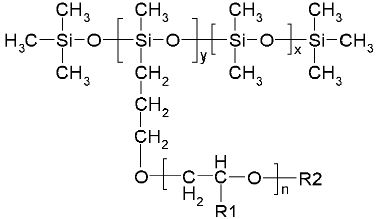 with R¹ being selected from hydrogen or methyl, R² being selected from H, C₁ to C₄ alkyl, or acetoxy, n = 1 to 50, x = 1 to 100, y = 1 to 20, the polyether-modified polydimethyl siloxane having a solubility in water of at least 9.1 wt.% and a surface tension in the range of 24 to 30 mN/m (dyn/cm).

Described is also a process of taking a dental impression the process comprising the steps of mixing the Base Paste and the Catalyst Paste described in the present text and claims to obtain a Mixed Paste, placing the Mixed Paste in contact with dental tissue, waiting until the Mixed Paste is hardened, removing the hardened composition from the dental tissue.

Described is also a process of producing the composition comprising the steps of providing the components of the Base Paste and Initiator Paste, mixing the components of the Base Paste and the Initiator Paste as described in the present text and claims.

Described is also a kit of parts comprising the composition described in the present text and claims and the following items alone or in combination: dental impression tray, application syringe, dental retraction material, and/or temporary crown and bridge material.

Unless defined differently, for this description the following terms shall have the given meaning:
By "paste" is meant a soft, viscous mass of solids dispersed in at least one liquid or a soft, viscous mass of a polymer.

A "particle" or "particulate filler" means a substance being a solid having a shape which can be geometrically determined. The shape can be regular or irregular. Particles can typically be analysed with respect to e.g. grain size and grain size distribution.

"Elastomeric" means rubber-elastic or rubber-like. Elastomeric materials can be characterized e.g. by a certain tensile strength and/or elongation at break. Other means for characterizing elastomeric materials include the measurement e.g. of the Young's modulus. Elastomeric materials typically have an E-modulus in the range from 0.8 to 10 MPa or from 1 to 8 MPa or from 1.5 to 6 MPa (determined e.g. according to DIN 53504, thickness of sample: 2 mm).

A "hardenable component or material" (e.g., "polymerizable component" or "crosslinkable component") is any component which can be cured or solidified e.g., by heating to cause polymerization, chemical crosslinking, radiation-induced polymerization or crosslinking by using a redox initiator. A hardenable component may contain, for example, only one, two, three or more polymerizable groups.

A "resin matrix" shall mean the organic part of the composition being composed of the hardenable components and organic diluents, if present.

An "initiator" is a substance or a group of substances being able to start or initiate or contribute to the hardening process of a hardenable compound.

The terms "vulcanizing", "hardening", "polymerizing", "crosslinking", "curing" and "setting" are used interchangeable and refer to compositions that have as a common attribute the development of a crosslinked polymer from relatively low molecular weight linear or branched polymers or pre-polymers by means of a chemical reaction that simultaneously forms these crosslinks and effectively extends chain length at room temperature.

The term "crosslinked polymer" refers to polymers that are the result of the reaction of the functional group or groups of the polymer chains or prepolymers that were lengthened or connected, e.g., to form a crosslinked network. In contrast to a thermoplastic polymer (i.e., a polymer that softens and flows upon heating) a crosslinked polymer, after crosslinking, is characteristically incapable of further flow.

The term "cationically polymerizable compound" is defined as a compound which can be polymerised using an initiator containing or being able to generate cations, especially reactive cations.

A "pre-polymer" is defined as a compound or a mixture of compounds obtainable by polymerization (such as e.g. polycondensation reaction) of monomers resulting in an intermediate product or mixture of products with increased molecular weight compared to the monomers used. The resulting intermediate product itself bears functional groups (either left over from the initial polymerization or introduced afterwards). The prepolymer containing functional groups can be used for further polymerization reactions (such as e.g. polycondensation reaction or polyaddition reaction) leading to a polymer or polymer mixture or a crosslinked polymer with increased molecular weight compared to the prepolymer.

"Aziridines" are a group of organic compounds sharing the aziridine functional group, which is a three membered heterocycle with one amine group and two methylene groups. The parent compound of the aziridines is called aziridine with molecular formula C₂H₅N.

An "alkyl-substituted aziridino group" is an aziridine group, wherein at least one of the hydrogen atoms of the methylene groups is substituted by an alkyl group, preferably by a C1 to C4 alkyl group, e.g. methyl, ethyl, n- and iso-propyl or n-, iso- or tert.-butyl group. In the chemical literature a "methyl substituted aziridine" is sometimes also referred to as "propylene imine".

"Polyether" or "polyether group containing compound" are compounds having a molecular weight of at least 150 g/mol and containing in the backbone at least 3, 10 or 20 ether moieties. Polyether containing compositions used as dental impression material can be cured by different mechanisms. Widely used is a crosslinking reaction using aziridine groups.

Examples of polyether and aziridino groups containing impression materials are given in US 5,569,691 (Guggenberger et al.), and US 5,130,348 (Zahler et al.). Commercially available materials are sold e.g. under the brand Impregum^{™} (3M Oral Care).

A "monomer" is any chemical substance which can be characterized by a chemical formula, bearing one or more polymerizable groups (including (meth)acrylate groups) which can be polymerized to oligomers or polymers thereby increasing the molecular weight. The molecular weight of monomers can usually simply be calculated based on the chemical formula given.

By "derivative" is meant a chemical compound showing a chemical structure closely related to the corresponding reference compound and containing all featured structural elements of the corresponding reference compound but having small modifications like bearing in addition comparably small additional chemical groups like e.g. CH₃, Br, Cl, or F or not bearing comparably small chemical groups like e.g. CH₃ in comparison to the corresponding reference compound. A derivative of a certain compound comprises the chemical structure of that compound but may contain other side groups or moieties.

The following examples might illustrate this: tetramethyl bis-phenol A bearing four additional methyl groups with respect to the reference compound bis-phenol A, and bis-phenol F not bearing two additional methyl groups with respect to the reference compound bis-phenol A are derivatives of bis-phenol A within the meaning of this definition.

"Room temperature curable" implies that the curing reaction can proceed at temperatures at or near 25°C. For example, the oral cavity of the mouth has an average temperature of approximately 32°C and is therefore near room temperature. Certain "high" temperature cured materials are designed to cure only at relatively high temperatures (e.g., >50°C or >100°C) and are stable (i.e., the curing reaction is retarded) at room temperature for prolonged periods. The compositions of the invention are room temperature vulcanizing.

A "dental composition" or a "composition for dental use" or a "composition to be used in the dental field" is any composition which can and is to be used in the dental field. In this respect the composition should not be detrimental to the patients' health and thus free of hazardous and toxic components being able to migrate out of the composition.

Dental compositions are typically hardenable compositions, which can be hardened at ambient conditions, including a temperature range of 15 to 50°C or 20 to 40°C within a time frame of 30 min or 20 min or 10 min. Higher temperatures are not recommended as they might cause pain to the patient and may be detrimental to the patient's health.

Dental compositions are typically provided to the practitioner in comparable small volumes, that is volumes in the range of 0.1 to 500 ml or 0.5 to 100 ml or 1 to 50 ml. Thus, the storage volume of useful packaging devices is within these ranges.

A "dental impression" may be described as an accurate representation of part or all of a person's dentition. It forms a "negative" of a person's hard dental tissue which can then be used to make a model (physical) of the dentition. This may be used for the fabrication of dentures, crowns or other prostheses. An impression is typically carried out by placing a viscous material into the mouth in a customised or stock tray. The material then sets to become an elastic solid, and when removed from the mouth retains the shape of the teeth and gingiva.

A "dental impression material" is a material used for making impressions of the tooth structure. A dental impression material is usually applied on a dental impression tray. A dental impression material can be based on different chemical substances and crosslink by various chemical reactions. Common materials used for dental impressions include alginate, agar, polyethers including aziridine substituted polyether materials as well as silicones, both condensation-cured silicones and addition-cured silicones including polyvinyl siloxanes (so-called VPS materials). The term "dental impression material" comprises precision impression materials, situation impression materials, bite registration materials, duplicating materials (applicable for the duplication of master models, e.g. for all-ceramic restorations requiring a refractory investment model and when inlays, onlays, cantilevers and other precision attachments are being fabricated) and modelling materials (applicable for e.g. reconstructing the gingival, producing crowns and bridges).

A "putty like dental impression material" is a kneadable dental impression material having a consistency of 35 mm or below according to ISO 4823:2015-08.

A "dental retraction material" is a material intended to be placed in the gingival sulcus, that is, the natural space between the hard dental tissue (i.e. tooth structure) and the gum tissue that surrounds the hard dental tissue. Once placed in the gingival sulcus, the dental retraction material will exert pressure on the surrounding tissue resulting in a widening of the gingival sulcus to enable the practitioner to get a more precise impression of the dental situation below the gum line during a dental impression process. Like a dental impression material, a dental retraction material is removed from the mouth of the patient after use.

The term "automixer-suitable material" relates to a multi-component material which can be dispensed, for example, from a two-component disposable cartridge through a static mixer, e.g., of SulzerMixpac Company (US 5,464,131, US 2001/0004082) or from tubular film bags in dual-chamber reusable cartridges through a dynamic mixer, e.g., in the "Pentamix^{™}", "Pentamix^{™} 2" and "Pentamix^{™} 3" devices of 3M Oral Care (cf. US 5,286,105 and US 5,249,862).

The term "dental tissue" includes the hard tooth substance (enamel and dentin), the gingival region (soft dental tissue) surrounding the hard tooth substance and hard tooth substance bearing orthodontic appliances.

A "temporary crown and bridge material" within the meaning of the invention is a hardenable material used for making dental crowns and bridges. These materials are typically used during the time period a dental technician needs for producing a permanent prosthetic work such as a crown or bridge. These time periods can last from a few days (1 to 6 days), a few weeks (1 to 4 weeks) or a few months (1 to 6 month).

A "surfactant" is an agent imparting wettability to a material, that is making the material more wettable compared to a material not containing a surfactant. The wettabilty can be determined by the water contact angle which can be measured using e.g. a goniometer DSA 10 (Krüss). A low water contact angle indicates a better wettability.

"Molecular weight" in the context of the invention and if not otherwise indicated always means number average molecular weight (Mₙ). The molecular weight (Mn) of the polymerizable compound before setting can be determined using nuclear magnetic resonance spectroscopy (end-group determination). In this respect proton (¹H) NMR techniques are employed to estimate the molecular weight of the precursor of the prepolymer. Integrated signals of the terminal -CH₂- groups are compared to the integrated sum of proton signals from backbone hydrocarbon protons taking into account comonomer ratio, if applicable. To achieve appropriate separation of terminal methylene proton signals from the backbone proton signals, terminal hydroxyl groups are esterified with trifluoroacetic acid.

"Ambient conditions" mean the conditions which the composition described in the present text is usually subjected to during storage and handling. Ambient conditions may, for example, be a pressure of 900 to 1100 mbar, a temperature of 10 to 40 °C and a relative humidity of 10 to 100 %. In the laboratory ambient conditions are typically in a range of 20 to 25 °C and 1000 to 1025 mbar.

A composition is "essentially or substantially free of" a certain component, if the composition does not contain said component as an essential feature. Thus, said component is not wilfully added to the composition either as such or in combination with other components or ingredient of other components. A composition being essentially free of a certain component usually does not contain that component at all. However, sometimes the presence of a small amount of the said component is not avoidable e.g. due to impurities contained in the raw materials used.

As used herein, "a", "an", "the", "at least one" and "one or more" are used interchangeably. Also herein, the recitations of numerical ranges by endpoints include all numbers subsumed within that range (e.g., 1 to 5 includes 1, 1.5, 2, 2.75, 3, 3.80, 4, 5, etc.).
Adding an "(s)" to a term means that the term should include the singular and plural form. E.g. the term "additive(s)" means one additive and more additives (e.g. 2, 3, 4, etc.).

Unless otherwise indicated, all numbers expressing quantities of ingredients, measurement of physical properties such as described below and so forth used in the specification and claims are to be understood as being modified in all instances by the term "about".

The terms "comprise" or "contain" and variations thereof do not have a limiting meaning where these terms appear in the description and claims. The term "comprise" shall include also the terms "consist essentially of" and "consist of".

Unless described otherwise, "wt.%" refers to the weight of the composition obtained when combining the compositions of the Base Paste and the Initiator Paste, which is also referred to as "whole composition".
Further, all references cited within the present text are herewith incorporated by reference.

### Detailed Description

Surprisingly it was found that the addition of certain polyether-modified polydimethyl siloxane(s) with a certain water-solubility to compositions comprising a cationically curable pre-polymer with a polyether backbone is suitable to address one or more of the object(s) outlined above.

It was also found that polyether-modified polydimethyl siloxane(s) having in addition a viscosity in a certain range are sometimes even more effective in addressing the object(s) outlined above.

Thus, a polyether-modified polydimethyl siloxane having a sufficiently high viscosity in combination with a sufficiently high water-solubility is sometimes preferred.

These polyether-modified polydimethyl siloxanes are in particular useful for improving the properties of a composition comprising cationically curable pre-polymers with a polyether backbone and at least two aziridino moieties, i.e. compositions which can be cured with the aid of a sulfonium salt containing initiator. Suitable initiators include in particular those described in US 2008/200585 A1 (Klettke et al.).

Further, it was found that mechanical properties such as tensile strength and elongation at break are typically not negatively affected.
In addition, by using the polyether-modified polydimethyl siloxanes described in the present text, properties such as the initial water-contact angle might also be improved.

The composition described in the present text is provided as a kit of parts comprising a Base Paste and an Initiator Paste. The two pastes are intended to be mixed before use to obtain a hardenable composition. The Base Paste contains a cationically curable pre-polymer.

The nature and structure of the cationically curable pre-polymer is not particularly limited unless the desired result cannot be achieved.

The cationically curable pre-polymer typically comprises a backbone and at least one or two reactive functional group(s).

The backbone of the cationically curable pre-polymer typically comprises moieties selected from polyether, polyester, polyurethane, silicone, polyalkylene, polystyrol, polysulfide and combinations thereof.

In the dental field a backbone containing polyether moieties can be preferred. Those groups typically also improve the hydrophilic properties of the composition.

According to one embodiment, the cationically curable pre-polymer includes a polyether group containing hardenable prepolymer as component (A) or part of component (A), that is, a prepolymer comprising a polyether group(s) and reactive moieties which upon addition of a suitable catalyst or initiator can react with each other and thus form a polymeric network.

The molecular weight (Mn) of the polyether group(s) containing pre-polymer is typically in a range of 150 to 20,000 g/mol, or in the range of 250 to 10,000 g/mol, determined e.g. with GPC methods know to the person skilled in the art.

Suitable polyethers or polyether groups, which can be used, include those which meet the requirements in terms of material properties with regard to the preferred use as dental materials.

Appropriate polyethers or polyether groups can be produced in a manner known to the person skilled in the art by the reaction of the starting compound having a reactive hydrogen atom with alkylene oxides, for example ethylene oxide, propylene oxide, butylene oxide, styrene oxide, tetrahydrofuran or epichlorohydrin or mixtures of two or more thereof.

Especially suitable are polyether compounds which are obtainable by polyaddition of ethylene oxide, 1,2-propylene oxide, 1,2-butylene oxide or tetrahydrofuran or of mixtures of two or more of the mentioned compounds with the aid of a suitable starting compound and a suitable catalyst.

The reaction products of low-molecular-weight polyfunctional alcohols having at least two hydroxyl groups with alkylene oxides, so-called polyethers, may also be used as polyols. The alkylene oxides preferably have from 2 to 4 carbon atoms. Suitable polyols are, for example, the reaction products of ethylene glycol, propylene glycol, butanediol or hexanediol isomers with one or more of the following alkylene oxides: ethylene oxide, propylene oxide or butylene oxides like tetrahydrofuran. Furthermore, the reaction products of polyfunctional alcohols such as glycerol, trimethylolethane or trimethylolpropane, pentaerythritol or sugar alcohols, or mixtures of two or more thereof, with the mentioned alkylene oxides, forming polyether polyols are also suitable.

Suitable starting compounds are, for example, water, ethylene glycol, 1,2- or 1,3-propylene glycol, 1,4- or 1,3-butylene glycol, 1,6-hexanediol, 1,8-octanediol, neopentyl glycol, 1,4-hydroxymethylcyclohexane, 2-methyl-1,3-propanediol, glycerol, trimethylolpropane, 1,2,6-hexanetriol, 1,2,4-butanetriol, trimethylolethane, pentaerythritol, mannitol, sorbitol, or mixtures of two or more thereof.

Especially suitable are polyether compounds as are obtainable by polyaddition of ethylene oxide, 1,2-propylene oxide, 1,2-butylene oxide or tetrahydrofuran or of mixtures of two or more of the mentioned compounds with the aid of a suitable starting compound and a suitable catalyst.

For example, polyether polyols which are prepared by copolymerisation of tetrahydrofuran and ethylene oxide in a molar ratio of 10:1 to 1:1, preferably 10:1 to 4:1, in the presence of strong acids, for example boron fluoride etherates, are suitable.

The composition comprises at least a cationically curable pre-polymer having at least 1 aziridine moiety or more, if desired, e.g. at least 2 or 3 or 4 or 5 or 6 aziridine moieties. Using a cationically curable pre-polymer with at least 2 aziridine moieties can be preferred to ensure a sufficient crosslinking.

According to another embodiment, the cationically curable pre-polymer comprises a polyether backbone and on average at least 2 aziridine moieties.

The term "on average" is to be interpreted such in the context of the present text that a mixture of a large number of compounds may comprise both compounds having less than 2 aziridine groups and also compounds having more than 2 aziridine groups although, when seen over the entirety of the compounds of component (A), the average functionality of all molecules is, with respect to aziridine groups, 2 or more.

All mentioned types of polyaddition or polycondensation products can be provided with aziridine groups by means of any desired subsequent reactions known to the person skilled in the art. For example, it is possible first to introduce, into an appropriate polymer, substituents which are in turn capable of reacting with suitable aziridine derivatives.

It is also possible to polymerise cyclic ethers, preferably epoxides, onto the chain so that products are obtained which at the end contain substituents which can react with aziridine. There come into consideration, for example, polyethers onto which halo-substituted epoxides, e.g. epibromohydrin, are polymerised.

Suitable possible methods for providing the polymers with aziridine groups are mentioned, e.g., in US 3,453, 242 (Schmitt et al.).

Suitable polymers carry the aziridine groups terminally or laterally, or terminally and laterally, but preferably terminally.

The aziridine groups containing compound typically has a dynamic viscosity η of 10 to 500 Pa*s, especially 15 to 300 Pa*s. A preferred viscosity range is 20 to 180 Pa*s at 23°C.

The aziridine equivalent is typically from 250 to 25,000 g/equivalent, especially 400 to 10,000 g/equivalent. The term "aziridine equivalent" is defined as (molecular mass of the molecule) / (number of aziridine groups present in the molecule).

Using compounds having such an aziridine equivalent weight may facilitate the provision of rubber-like or elastomeric materials (after hardening). Compounds having an aziridine equivalent weight outside this range might either be too hard or brittle or too soft, e.g. do not have the desired Shore hardness or tensile strength.

The cationically curable pre-polymer which can be used may comprise only one type of aziridine group containing polymer. It is, however likewise possible for the cationically hardenable compound to comprise two or more different types of aziridine polymers, for example 3, 4 or 5 different types.

A "type of polymer" is understood, in the context of the present invention, to be a polymer as results from the polyaddition or polycondensation of selected monomers under the selected reaction conditions. A type of polymer can accordingly include polymer molecules of differing chemical constitution and differing molecular weight, depending on the reaction conditions selected. However, two reactions carried out using identical monomer compositions under identical reaction conditions always result, in accordance with the invention, in identical types of polymer. Two reactions which are carried out using identical monomers but under different reaction conditions may result in identical types of polymers but need not do so. The crucial factor therein is whether there are identifiable differences - in terms of chemical constitution, molecular weight and further parameters which can be determined - that are of relevance to the material properties. Two reactions which are carried out using different monomer compositions always result, in accordance with the invention, in different types of polymers.
Reactive side groups which pending from or attached to the backbone of the prepolymer include those characterized by the following formula 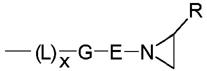 wherein R represents H, C₁-C₁₂ alkyl, C₂-C₁₂ alkenyl, C₂-C₁₂ alkinyl, C₇-C₁₅ alkylaryl, C₇-C₁₅ arylalkyl, C₃-C₁₂ cycloalkyl, and wherein hydrogen atoms may be replaced by Cl or F and/or wherein up to five carbon atoms may be replaced by atoms or group of atoms selected from O, CO, N, S,

E represents a C₁ - C₁₈ branched or unbranched hydrocarbon chain wherein up to five carbon atoms may be replaced by atoms or group of atoms selected from O, CO, N, S,

G represents a group selected from C(O)O, C(O)NR, C(O), C(O)C(O), C(O)(CH₂)ₘC(O) with m = 1 to 10, C(S)NR, CH₂,
L represents O, S, NR with x = 0 or 1.

It can be preferred, if the cationically curable pre-polymer has a linear molecular structure. Thus, the cationically curable pre-polymer may typically comprise a linear backbone, which is typically end-capped with aziridine groups. Usually, there are no side chains, especially cationically hardenable side chains pending from the backbone.

The cationically curable pre-polymer is typically present in an amount, which allows the formation of a sufficiently crosslinked network, in order to fulfil the practitioners needs.

By varying the amount of the cationically curable pre-polymer, e.g. the viscosity and the hardness of the cured composition can be adjusted.

If the amount of the cationically curable pre-polymer is too low, the resulting composition might not cure within the desirable period of time or might show not desirable mechanical properties.

If the amount of the cationically curable pre-polymer is too high, the resulting composition might be too viscous.

If desired, besides the cationically curable pre-polymer containing at least two aziridine groups, further curable compounds can be present being different from the cationically hardenable compound described above.

Thus, blends of various cationically polymerizable pre-polymers are also contemplated in the present text. Examples of such blends include two or more weight average molecular weight distributions of resin-containing compounds, such as low molecular weight (below 200), intermediate molecular weight (200 to 10,000) and higher molecular weight (above 10,000).

The cationically curable pre-polymer is typically present in the following amounts:
Lower amount: at least 5 or 10 or 15 wt.%;
Upper amount: at most 80 or 75 or 70 wt.%;
Range: 5 to 80 or 10 to 75 or 15 to 70 wt.%,
wt.% with respect to the weight of the whole composition.

The Base Paste also contains filler(s).

The BET surface of the filler is typically in a range of 0.05 to 50 m²/g or 0.5 to 30 m²/g or 0.5 to 20 m²/g. Using a filler with a BET surface within this range can be beneficial to adjust the viscosity and tensile strength.

If desired, the BET surface of the filler can be determined as described in DIN 66132. Alternatively, the values for the BET surface are taken from a material data sheet provided by the supplier.

The size of the filler particles should be such that a homogeneous mixture can be obtained. The particle distribution is preferably chosen such that there are no fillers with particle sizes of more than 50 µm.

Typically, the size of the filler particles (d50 value) is below 40 µm or below 10 µm or below 5 µm. Typical ranges (d50 value) include 0.1 to 40 µm or 0.5 to 20 µm or from 1 to 10 µm.

If the filler particles are too small, the viscosity of the resulting composition might increase to a not desirable limit.

If the filler particles are too big, the detail accuracy might be negatively affected.

The surface of the filler can be surface treated, if desired. A surface treatment can improve the miscibility of the filler with the other components present in the composition. A "non-surface treated filler" is a filler having a surface which has not been exposed to reactive substances resulting in a modification of the surface of the filler to make the filler more compatible with other components of the composition.

Reinforcing and non-reinforcing fillers can be used, as desired. A combination of reinforcing and non-reinforcing fillers can be preferred.

Examples of reinforcing fillers include fumed silica, carbon black and the like. They also can improve mechanical properties like tensile strength or tear strength, of the cured composition.

The amount of reinforcing fillers can range from 0 to 10 wt.%, in particular from 0.5 to 7 wt.% with respect to the whole composition.

Examples of non-reinforcing fillers include inorganic, hydrophilic or hydrophobic fillers such as silica, alumina, magnesia, titania, zirconium silicate, inorganic salts (e.g. barium sulphate, calcium carbonate), plaster, quartz, cristobalite, kaolin, talcum, feldspar, bentonite, wollastonite, nephelinsyenit, zeolite, molecular sieves such as sodium aluminium silicate, silicates and glasses.

The following fillers were found to be particularly useful: quartz comprising amino-silane groups (e.g. Silbond^{™} 600 AST, Silbond^{™} 800 AST; Quarzwerke Frechen), wollastonite comprising amino-silane groups (e.g. Tremin^{™} 283-600 AST or Tremin^{™} 939-300 AST; Quarzwerke Frechen), quartz/kaolin mixture comprising amino-silane groups (e.g. Aktisil^{™} AM; Quarzwerke Frechen) and quartz comprising epoxy groups (e.g. Silbond^{™} 600 EST, Silbond^{™} 800 EST; Quarzwerke Frechen).

The amount of non-reinforcing fillers can range from 1 to 70 wt.%, in particular from 5 to 50 wt.% with respect to the whole composition.

Mixtures of silicone dioxides, such as a diatomaceous earth and/or fumed silica are sometimes preferred. Those filler are commercially available from companies like Cabot Corporation, Wacker or Degussa under the trade names Aerosil^{™} (Degussa) HDK^{™}-H (Wacker), Cab-o-Sil^{™} (Cabot).

Filler(s) are also present in the Initiator Paste.

The Initiator Paste may contain the same kind or different kinds of fillers compared to the Base Paste.

Filler is typically present in the following amounts:
Lower amount: at least 5 wt.% or at least 7,5 wt.% or at least 10 wt.%;
Upper amount: at most 80 wt.% or at most 75 wt.% or at most 70 wt.%;
Range: 5 to 80 or 7.5 to 75 or 10 to 70 wt.%;
wt.% with respect to the whole composition.

With respect to the Base Paste and/or the Initiator Paste, filler is typically present in the following amounts:
Lower amount: at least 1 or 5 or 10 wt.%;
Upper amount: at most 70 or 60 or 50 wt.%;
Range: 1 to 70 or 5 to 60 or 10 to 50 wt.%.

If the amount of the filler is too low, a desired Shore hardness and/or tensile strength might not be obtained.

If the amount of the filler is too high, the elasticity of the cured composition might negatively be affected and the viscosity of the un-cured composition might be too high. Moreover, the shelf life might negatively be influenced.

The Initiator Paste contains an initiator which is able to start the hardening of the cationically curable pre-polymer comprising a polyether backbone and at least two reactive functional moieties contained in the Base Paste.

The nature and structure of the initiator is not particularly limited unless the desired result cannot be achieved.

According to one embodiment, the initiator is water-soluble.

Using a water-soluble initiator component can be beneficial, as the initiator will start to dissolve upon contact with water being present in the mixed composition and thus become more effective. There is no need for adding a further dissolvent for dissolving a water-soluble initiator.

According to one embodiment, the initiator is selected from Lewis acids or Broensted acids or precursors of Lewis acids which can be activated by radiation to produce a Lewis acid. In principle both organic and inorganic acids can be used.

Specific examples of Broensted and Lewis acids, which can be used, include sulfonic acids, phosphonic acids, phosphoric acids, carboxylic acids, antimonic acids, boric acids and mixtures and salts thereof.

Particular useful initiators include sulfonium salts, especially alkyl sulfonium salts or sulfonium salts derived from glutaconic acid. Those and others are described e.g. in US 4,167,618 (Schmitt et al.) and US 2003/0153726 A1.

Suitable sulfonium acids and salts thereof include 4-toluenesulphonic acid, 4-phenolsulphonic acid, 4-bromobenzenesulphonic acid, 4-chlorobenzenesulphonic acid, benzenesulphonic acid, alkylbenzenesulphonic acids, in particular dodecylbenzenesulphonic acid, naphthalene-2-sulphonic acid and alkanesulphonic acids.

Trialkylsulfonium salts as are described in, e.g., US 4,167,618 (e.g.: col 2, line 36 to col 4, line 32 and examples) are especially suitable as initiator substances.

The following initiator compounds were found to be especially useful: zinc salt of p-toluenesulfonic acid, □-(S-lauryl-S-ethylsulfonium)butyronitrile tetrafluoroborate, dodecylbenzenesulfonic acid zinc salt, □-(S-lauryl-S-ethylsulfonium)-□-phenylacrylic acid butyl ester tetrafluoroborate.

A further preferred class of initiators can be classified as sulfonium salts or derivatives of glutaconic acid esters as described in US 2008/0200585 A1 (Klettke).

These initiators comprise at least one structural element of the following formula: 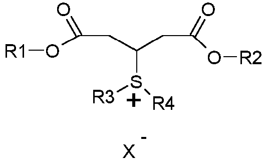 wherein
X⁻ is a non or low coordinating anion,
R1, R2, R3 and R4 are independently linear, cyclic or branched C₁ - C₂₀ alkyl or alkylene groups, wherein one or more of the methylene groups may be substituted by - CO- , -CONH-, -CON(CH₃)-, -S- and/or -O-,
and wherein R1, R2, R3 and/or R4 can act as a bridging element, connecting two or more structural elements.

The term "non or low coordinating group" includes anions of strong acids, preferably acids having a pKs value below 2. Respective examples are BF₄⁻, CF₃SO₃⁻, SbF₆⁻, AsF₆⁻ or 2,5-di-chloro-benzolsulfonate, but even other low coordinating anions can be used.

The term "bridging element" is defined as a chemical group being able to connect two or more of the aforementioned structural elements comprising at least one sulfonium group. Examples of bridging elements include -(CH₂)₈-, -(CH₂)₆- or -(CH₂)₄- moieties.

Other initiators which can be used include strong acids such as hexafluoroantimonic acid, hexafluorophosphoric acid or tetrafluoroboric acid.

The use of phosphonic acids such as vinylphosphonic acid and propylphosphonic acid is also possible.

Polymeric acids such as polyvinylphosphonic acid, polyacrylic acid, copolymeric acids, prepared from maleic anhydride with other monomers can also be used, if desired.

Furthermore, saturated and unsaturated carboxylic acids such as propionic acid, succinic acid, tartaric acid, trimellitic acid, benzoic acid, phenylacetic acid, citric acid, maleic acid, adipinic acid, o-chlorobenzoic acid or reaction products of polyvalent alcohols and acid anhydrides such as maleic anhydride and succinic anhydride can also be used.

Those and other starters are described e.g. in US 2003/153726 (Eckhardt et al.).

The molar ratio between the initiator and the cationically curable pre-polymer curable by a ring-opening reaction includes ranges of 1:0.1 to 1:20, or 1:0.5 to 1:10, or 1:0.8 to 1: 30.

As the initiator does not only act as a catalyst but chemically react to a certain extend with the hardenable composition, a sufficient amount of initiator should be present.

The amount of the initiator to be used is not particularly limited, unless the desired curing reaction cannot be initiated.

If the amount of initiator is too low, the desired viscosity and/or consistency may not be obtained.

If the amount of initiator is too high, the resulting composition might not be sufficiently homogenous and the ability to homogenously mix the components might be negatively affected.

The initiator is typically present in the following amounts:
Lower amount: at least 1 or 2 or 3 wt.%;
Upper amount: at most 30 or 20 or 10 wt.%;
Range: 1 to 30 or 2 to 20 or 3 to 10 wt.%;
wt.% with respect to the weight of the whole composition.

The Initiator Paste also contains a diluent.

The chemical nature and the physical properties of the diluent are not particularly limited.

Suitable diluting agent(s) usually do not contain reactive moieties like -SH or -COOH, primary or secondary amino groups, but may contain -OH.

Suitable diluents or plasticisers include C₁₂-C₁₅ alkyl acetates or lactates, C₂- to C₂₂-dialkyl esters of C₂- to C₆-dicarboxylic acids such as bis(2-ethylhexyl) adipate, dioctyl maleate, diisopropyl adipate, esters of phthalic acid with branched alcohols like bis(2-ethylhexyl)phthalate or polymeric phthalates, ethyl or butyl esters of citric acid or of acetylcitric acid, aromatic components such as poly phenyls, dibenzyl toluene, xylyl toluene, dixylyl toluene, polyphenyls, including wax-like polyphenyls, isomeric mixtures of C₂₀ to C₄₀ aromatic compounds and polymeric components such as polyethers, polyesters, polycarbonates, polytetrahydrofuranes, polyolefines, aromatic and aliphatic sulfonic acid esters such as C₂- to C₂₀-alkylsulfonic acid esters of phenol or of C₁- to C₂₂-alkanols and mixtures thereof.

A mixture of diluents or plasticisers of the ester type and aromatic type is sometimes preferred. An example of a preferred diluent and/or plasticiser combination is a mixture of acetyl tributyl citrate and dibenzyl toluene.

Examples of useful diluents also include those according to the following formula:

R¹-O-[CH₂CH₂O]ₙ-[R²O]ₘ-R³

wherein R¹ represents hydrogen or an aromatic or aliphatic, linear or branched hydrocarbon group having 1-20 carbon atoms, R² represents an alkylene having 3 carbon atoms, R³ represents hydrogen or a C₁-C₃ alkyl group, n has a value of 0 to 40, m has a value of 0 to 40 and the sum of n+m being at least 2.

It will be understood that in the above formula, the units indexed by n and m may appear as blocks or they may be present in an alternating or random configuration. Examples of non-ionic surfactants according to the formula above include alkylphenol oxethylates such as ethoxylated p-iso-octylphenol commercially available under the brand name TRITON^{™} such as for example TRITON^{™} X 100 wherein the number of ethoxy units is 10 or TRITON^{™} X 114 wherein the number of ethoxy units is 7 to 8.

Still further examples include those in which R¹ in the above formula represents an alkyl group of 4 to 20 carbon atoms, m is 0 and R³ is hydrogen. An example thereof includes isotridecanol ethoxylated with 8 ethoxy groups and which is commercially available as GENAPOL^{™}X080 from Clariant GmbH.

Diluents according to the above formula with R¹ and R³ representing a C₁-C₃ alkyl chain or hydrogen and in which the hydrophilic part comprises a block-copolymer of ethoxy groups and propoxy groups may be used as well.

Such non-ionic surfactants are commercially available from Clariant GmbH under the trade designation GENAPOL^{™} PF 40 and GENAPOL^{™} PF 80. Further suitable non-ionic surfactants that are commercially available include Tergitol^{™} TMN 6, Tergitol^{™} TMN 10, or Tergitol^{™} TMN 100X.

Also statistical, alternating or block copolymers of ethylene oxide and propylene oxide are suitable according to the present invention. Such components are available e.g. under the trade name Breox^{™} A, Synperonic^{™} or Pluronic^{™}.

If desired, diluents may also be present in the Base Paste, wherein the diluents present in the Base Paste may be different or identical to those being present in the Initiator Paste.

The diluent is typically present in the following amounts:
Lower amount: at least 2 wt.% or at least 2.2 wt.% or at least 2.5 wt.%;
Upper amount: at most 80 wt.% or at most 75 wt.% or at most 70 wt.%;
Range: 2 to 80 or 2.1 to 75 or 2.5 to 70 wt.%;
wt.% with respect to the whole composition.

The composition described in the present text comprises at least one polyether-modified polydimethyl siloxane. This component has the function of a surfactant.

The polyether-modified polydimethyl siloxane can be present in the Base Paste or the Initiator Paste or in both pastes, the Base Paste and the Initiator Paste, wherein the presence of the polyether-modified polydimethyl siloxane in the Base Paste is sometimes preferred.

The composition described in the present text comprises a polyether-modified polydimethyl siloxane of the following formula: 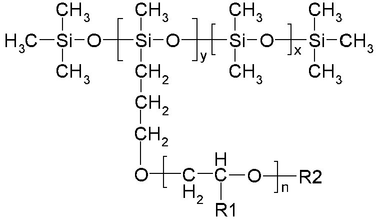 with
R¹ being selected from hydrogen or methyl,
R² being selected from H, C₁ to C₄ alkyl, or acetoxy,
n = 1 to 50 or 1 to 30 or 1 to 20,
x = 1 to 100, y = 1 to 20,
wherein the polyether-modified polydimethyl siloxane has a water solubility of at least 9.1 wt.%.

Polyether-modified polydimethyl siloxanes according to the above formula where R¹ is H (ethoxy moieties) are sometimes preferred.

The polyether-modified polydimethyl siloxane described in the present text has a surface tension in the range of 24 to 30 or 25 to 28 dyn/cm (in de-ionized water at 25°C for a concentration of 1%) The surface tension is determined according to DIN EN 14370 (Nov. 2004).

In contrast to other surfactants (such as PEG-8 methicone or Silwet^{™} L-77) the surface tension of the polyether-modified polydimethyl siloxane described in the present text is higher.

The polyether-modified polydimethyl siloxane has a water-solubility of at least 9.1, or at least 10 or at least 20 or at least 30, or at least 50 or at least 100 wt.% with respect to water. According to a further embodiment, the polyether-modified polydimethyl siloxane is miscible with water without phase separation even at a concentration of more than 1 g/ml.

If the water solubility is too low the desired decrease of water contact angle may not be satisfactory.

According to a further embodiment, the water solubility is at least 10 wt.% and n is selected from 3 to 50 or 3 to 30 or 3 to 20.

The water solubility is determined as described in the example section.

Besides the water solubility, the viscosity of the polyether-modified polydimethyl siloxanes may have an impact on the properties of the composition described in the present text.

Suitable polyether-modified polydimethyl siloxanes typically have a viscosity of at least 100 cSt, or at least 150 cSt, or at least 200 cSt. The viscosity is typically within a range of 100 cSt (100 mPa*s) to 500 cSt (500 mPa*s) or 150 cSt (150 mPa*s) to 400 cSt (400 mPa*s).

If the viscosity is too low, the molecular weight of the polyether-modified polydimethyl siloxane may be too low, too. This may increase the possibility that the polyether surfactant migrates from the composition during and/or after the hardening process.

If the viscosity is too high, the molecular weight of the polyether-modified polydimethyl siloxane may be too high, too. This may increase the possibility that the flow properties of the composition are reduced.

According to one embodiment, the polyether-modified polydimethyl siloxane has a water solubility of at least 10 wt.% and a viscosity in the range of 100 to 500 cSt or a water solubility of at least 30 wt.% and a viscosity in the range of 150 to 400 cSt.

The viscosity is determined as described in the examples section.

The polyether-modified polydimethyl siloxane described in the present text is typically present in the following amounts:
Lower amount: at least 0.05 or 0.1 or 0.5 wt.%;
Upper amount: at most 10 or 8 or 5 wt.%;
Range: 0.05 to 10 or 0.1 to 8 or 0.5 to 5 wt.%.
wt.% with respect to the amount of the whole composition.

If too much of the polyether-modified polydimethyl siloxane is present in the composition, the possibility that the polyether-modified polydimethyl siloxane migrates out of the composition either in its uncured or cured state increases. Further, the possibility that the cured composition starts to dissolve, if stored in water, may increase as well.

Besides the polyether-modified polydimethyl siloxane described above, the composition may comprise further surfactants.

Those other surfactants can be present in the Base Paste or the Initiator Paste or in both pastes, the Base Paste and the Initiator Paste, wherein the presence of the surfactant in the Base Paste is sometimes preferred.

Useful surfactants include polyether carbosilanes of the following formula:

Q-P-(OCₙH₂ₙ)ₓ-OZ

in which Q stands for R₃Si- or R₃Si-(R'-SiR₂)ₐ-R'-SiR''₂- , where every R in the molecule can be the same or different and stands for an aliphatic C₁-C₁₈, a cycloaliphatic C₆-C₁₂ or an aromatic C₆-C₁₂ hydrocarbon radical, which can optionally be substituted by halogen atoms, R' is a C₁-C₁₄ alkylene group, R" is R in the case of a≠0 or is R or R₃SiR' in the case of a=0, and a=0-2; P stands for a C₂-C₁₈ alkylene group, preferably a C₂-C₁₄ alkylene group or A-R‴, where A represents a C₂-C₁₈ alkylene group and R‴ a functional group selected from: -NHC(O)-, -NHC(O)-(CH₂)ₙ₋₁-, -NHC(O)C(O)-, -NHC(O)(CH₂)ᵥC(O)-, - OC(O)-, -OC(O)-(CH₂)ₙ₋₁-, -OC(O)C(O)-, -OC(O)(CH₂)ᵥC(O)-, - OCH₂CH(OH)CH₂OC(O)(CH₂)ₙ₋₁-, -OCH₂CH(OH)CH₂OC(O)(CH₂)ᵥC(O)- with v=1-12; Z is H or stands for a C₁-C₄ alkyl radical or a C₁-C₄ acyl radical; x stands for a number of 1 to 200 and n stands for an average number from 1 to 6, preferably 1 to 4. Thus, the element -SiR''₂-can also comprise the substructure -Si(R)(R₃SiR')-.

The composition described in the present text may also comprise in addition to other ingredients and surfactants, alone or in combination an F-containing component including those described in EP 2 442 778 B1 (3M), especially those described on pages 12 to 16, paragraphs [0137] to [0162].

Useful F-containing components may be described by the following formula:

(G¹-L¹-O)ₛ-R_{f}^{a}-O-L²-G²

wherein:
G¹ and G² each independently represents a non-ionic endgroup that is free of polyoxyalkylene groups or contains polyoxyalkylene such that the total amount thereof in the F-containing compound is not more than 10 wt.% based on the molecular weight of the F-containing compound;
L¹ and L² each independently represents an aliphatic hydrocarbon group or a partially or fully fluorinated aliphatic hydrocarbon group;
R_{f}^{a} represents a mono-valent or divalent partially or fully fluorinated aliphatic group or a partially or fully fluorinated aliphatic group interrupted by one or more oxygen atoms;
with the proviso that at least one of the following conditions is fulfilled:
   (i) at least one of the moieties L¹-G¹ and L²-G² is partially or fully fluorinated or
   (ii) R_{f} is a partially or fully fluorinated aliphatic group interrupted by one or more oxygen atoms.

More precisely, the F-containing component may be described by the following formula:

T₁-X-[(O-CF₂-CF₂)ᵤ-(O-CF₂)ᵥ-(O-CF(CF₃)-CF₂)_{w}-(O-CF₂-CF₂-CF₂)ₓ-O]-X-T₂
with u = 0 to 8, v = 0 to 8, w = 0 to 8 and x =0 to 8 and u+v+w+x ≥ 1,
wherein T₁ and T₂ can be equal or different and are independently selected from -COOR, -CONR^{b}R^{c} -CH₂OH, -CF₂OR, -CHFOH, -CHFOR, -CH₂OR or -F with R and being a linear or branched alkyl rest (C₁ to C₉), aryl rest (C₁ to C₉) or alkylaryl rest (C₁ to C₉) each of which may optionally be substituted with one or more substituents selected from the group consisting of hydroxyl, amino group, halogen atom, an SiH group and a group capable of reacting with SiH, R^{b} and R^{c} independently representing H or having a meaning as given for R, and wherein X is selected from -(CF₂)₁₋₆-, -CF(CF₃)- and -CHF-CF₂-.

Particular examples of the F-containing component include: CF₃-O-CF₂-O-CF₂-CF₂-O-CHF-T, CF₃-(O-CF₂)₂-O-CF₂-CF₂-O-CHF-T, CF₃-(O-CF₂)₃-O-CF₂-CF₂-O-CHF-T, CF₃-O-CF₂-O-CF₂-CF₂-O-CHF-CF₂-T, CF₃-(O-CF₂)₂-O-CF₂-CF₂-O-CHF-CF₂-T, CF₃-(O-CF₂)₃-O-CF₂-CF₂-O-CHF-CF₂-T, C₃F₇-O-CF₂-CHF-T, CF₃-O-CF₂-O-CF₂-CF₂-O-CF₂-CHF-T, CF₃-(O-CF₂)₂-O-CF₂-CF₂-O-CF₂-CHF-T, CF₃-(O-CF₂)₃-O-CF₂-CF₂-O-CF₂-CHF-T, C₃F₇-O-CF₂-CHF-CF₂-T, CF₃-O-CF₂-CF₂-CF₂-O-CF₂-CHF-CF₂-T, CF₃-O-CF₂-O-CF₂-CF₂-O-CF₂-CHF-CF₂-T, CF₃-(O-CF₂)₂-O-CF₂-CF₂-O-CF₂-CHF-CF₂-T, CF₃-(O-CF₂)₃-O-CF₂-CF₂-O-CF₂-CHF-CF₂-T, CF₃-(O-CF₂)₃-O-CF₂-T, CF₃-(O-CF₂)₅-O-CF₂-T, C₂F₅-(O-CF₂-CF₂)₁-O-CF₂-T, C₃F₇-(O-CF₂-CF₂)₁-O-CF₂-T, C₄F₉-(O-CF₂-CF₂)₁-O-CF₂-T, C₂F₅-(O-CF₂-CF₂)₂-O-CF₂-T, CF₃-(O-CF₂-CF₂)₂-O-CF₂-T, C₃F₇-(O-CF₂-CF₂)₂-O-CF₂-T, C₄F₉-(O-CF₂-CF₂)₂-O-CF₂-T, CF₃-(CF₂)₂-(O-CF(CF₃)-CF₂)₂-O-CF(CF₃)-T, CF₃-(CF₂)₂-(O-CF(CF₃)-CF₂)₃-O-CF(CF₃)-T, CF₃-(CF₂)₂-(O-CF(CF₃)-CF₂)₄-O-CF(CF₃)-T, CF₃-(CF₂)₂-(O-CF(CF₃)-CF₂)₅-O-CF(CF₃)-T, CF₃-(CF₂)₂-(O-CF(CF₃)-CF₂)₆-O-CF(CF₃)-T, CF₃-CFH-O-(CF₂)₃-T, CF₃-CFH-O-(CF₂)₅-T, CF₃-CF₂-O-(CF₂)₃-T, CF₃-CF₂-O-(CF₂)₅-T, Rf-(O-CF₂-CF₂-CF₂)ₙ-O-CF₂-CF₂-T, with n = 1 to 25 and Rf being a linear or branched per- or partly fluorinated alkyl rest (C1 to C6), wherein the alkyl chain can be interrupted by O atoms, T-CF₂-O-(CF₂-CF₂-O)ₚ-(CF₂-O)_{q}-CF₂-T, with p/q = 0.5 to 3.0 and an molecular weight in the range of 500 to 4,000 g/mol, T-CF₂-(O-CF(CF₃)-CF₂)ₙ-(O-CF₂)ₘ-O-CF₂-T, with n/m = 20 to 40 and a molecular weight in the range of 650 to 3,200 g/mol, and mixtures thereof, wherein T can be equal or different and are independently selected from -COOR, -CONR^{b}R^{c} -CH₂OH, -CF₂OR, -CHFOH, -CHFOR, -CH₂OR or -F with R and being a linear or branched alkyl rest (C₁ to C₉), aryl rest (C₁ to C₉) or alkylaryl rest (C₁ to C₉) each of which may optionally be substituted with one or more substituents selected from the group consisting of hydroxyl, amino group, halogen atom, an SiH group and a group capable of reacting with SiH, R^{b} and R^{c} independently representing H or having a meaning as given for R, and wherein X is selected from -(CF₂)₁₋₆-, -CF(CF₃)- and -CHF-CF₂-.

Suitable fluorinated compounds also include fluorinated polyethers that are commercially available under the tradename FOMBLIN^{™}, GALDEN^{™} and H-Galden^{™}, Fluorolink^{™} materials which may be prepared using preparation methods described in US2007/0276068, EP 870877, WO 2004/060964, WO 2007/140091, US 2007/015864, US 2007/015864, US 2007/025902 and US 2007/015937.

HFPO can be obtained as described in US 3,242,218 or US 2004/0124396. The general formula of a methyl ester derivative of HFPO is C₃F₇O[CF(CF₃)CF₂O]ₙCF(CF₃)COOCH₃ with n being 1 to 8. HFPO-Amidol as described in WO2004/060964 A1 has the formula CF₃-(CF₂)₂-(O-CF(CF₃)-CF₂)_{z}-O-CF(CF₃)-CONHCH₂CH₂OH.

The use of HFPO and/or HFPO-Amidol in combination with the polyether-modified polydimethyl siloxane described in the present text is sometimes preferred.

The composition described in the present text can also comprise one or more additives.

Suitable additives include pigment(s), flavour(s), stabilizer(s), retarder(s), astringent(s), triacyl esters of glycerol, and other ingredients well known to those skilled in the art.

All kinds of known and compatible softeners and rheology modifiers like nonreactive polymeric fluids or fats commonly used in commercialized dental impression materials can be added.

Preferred are those ingredients and additives that do not add unpleasant smell or taste. Compounds that have an unpleasant smell might be removed by thinfilm evaporation, if needed.

The amounts and types of each ingredient in the composition should be adjusted to provide the desired physical and handling properties before and after polymerization. For example, the polymerization rate, polymerization stability, fluidity, compressive strength, tensile strength and durability of the dental material typically are adjusted in part by altering the types and amounts of polymerization initiator(s) and, if present, the loading and particle size distribution of filler(s). Such adjustments typically are carried out empirically based on experience with dental materials of the prior art.

Examples for pigments include titanium dioxide or zinc sulphide (lithopones), red iron oxide 3395, Bayferrox^{™} 920 Z Yellow, Neazopon^{™} Blue 807 (copper phthalocyanine-based dye) or Helio^{™} Fast Yellow ER. If present, pigments are typically present in an amount of 0.01 to 3 wt.% with respect to the whole composition.

Further additives, which can be added, include stabilizers, especially free radical scavengers such as substituted and/or unsubstituted hydroxyaromatics (e.g. butylated hydroxytoluene (BHT), hydroquinone, hydroquinone monomethyl ether (MEHQ), 3,5-di-tert-butyl-4-hydroxyanisole (2,6-di-tert-butyl-4-ethoxyphenol), 2,6-di-tert-butyl-4-(dimethylamino)methylphenol, 4-methoxybenzylalcohol, 2,6-di-tert.-butyl-4-methylphenol (Ionol^{™}), 3-methoxyphenol or 2,5-di-tert-butyl hydroquinone, 2-(2'-hydroxy-5'-methylphenyl)-2H-benzotriazole, 2-(2'-hydroxy-5'-t-octylphenyl)-2H-benzotriazole, 2-hydroxy-4-methoxybenzophenone (UV-9), 2-(2'-hydroxy-4',6'-di-tert-pentylphenyl)-2H-benzotriazole, 2-hydroxy-4-n-octoxybenzophenone, 2-(2'-hydroxy-5'-meth-acryloxyethylphenyl)-2H-benzotriazole, phenothiazine, tocopherol, polyethylene imine, substituted pyridines (e.g. 2,6-di-tert.-butyl-4-methylpyridine) and HALS (hindered amine light stabilizers). If present, stabilizers are typically present in an amount of 0.01 to 3 wt.% with respect to the whole composition.

Retarder(s) which may be added include amine or alkaline retardants, in particular, 1-aryl- and 1-alkyl-substituted imidazoles.

Examples of retarders which can be used include 1-methylimidazole, 1-(n-butyl)imidazole, 1-decylimidazole, 1-laurylimidazole, 1-omega-bis(1-imidazolyl)-C₁-C₁₀ alkanes such as 1,2-bis(1-imidazolyl)ethane and 1,10-bis(1-imidazolyl)decane, 11-(1-imidazolyl)undecanoic acid benzylamide, 1-cyclohexylimidazole, 1-benzylimidazole, 1-(2-ethoxyethyl)imidazole, 1-(4-methoxyphenyl)imidazole, and 1-[3-(2-ethylhexanoyl)amidopropyl]-imidazole. Suitable retarders are also described in US 4,532,268 (Jochum et al).

If present, retarders are typically present in an amount of 0.01 to 3 wt.% with respect to the whole composition.

Astringent(s) which may be included comprise aluminum salts like aluminum sulfate, aluminum ammonium sulfated, aluminum chlorohydrate, aluminum acetate and mixtures thereof. Useful astringent(s) can also contain iron or manganese containing substances.

Incorporating an astringent may help to prevent or reduce the risk of bleeding during use or after removal of the composition from the mouth of a patient.

If present, astringents are typically present in an amount of 0.01 to 3 wt.% with respect to the whole composition.

Likewise, suitable as additives are triacyl esters of glycerol of non-animal origin. Suitable additives can consist of, for example, modified fats of vegetable origin such as hydrogenated palm oil or soybean oil or synthetic fats.

Suitable fats are described in US 6,395,801. Avocado oil, cottonseed oil, groundnut oil, cocoa butter, pumpkin seed oil, linseed oil, maize germ oil, olive oil, palm oil, rice oil, rapeseed oils, safflower oil, sesame oil, soybean oil, sunflower oil, grapeseed oil, wheat germ oil, Borneo tallow, fulwa butter, hemp oil, illlipé butter, lupin oils, candlenut oil, kapok oil, katiau fat, kenaf seed oil, kekuna oil, poppy seed oil, mowrah butter, okra oil, perilla oil, sal butter, shea butter and tung oil are especially suitable, provided that the fats in question have been hydrogenated before use. Suitable hydrogenated fats are considered to be those whose iodine value is less than 20 (measured in accordance with the DGF [German Society for Fat Science] standard C-V 11 Z2). Fat hydrogenation procedures are described, for example, in "Ullmanns Enzyklopädie der industriellen Chemie", 4th edition, volume 11, p. 469.

Mixtures of naturally occurring fats, and also synthetically prepared fats such as Softisan^{™} 154 or Dynasan^{™} 118 (from Hüls Comp.) can likewise be used. The preparation of such synthetic triacyl glycerides is known to the person skilled in the art and can be carried out by starting from glycerol and the appropriate fatty acid methyl esters.

Preferred triacyl glycerides correspond to the following formula:

R²-O-CH₂-CH(OR¹)-CH₂-O-R³

in which R¹, R² and R³ denote, each independently of the others, C₁₁H₂₃CO, C₁₃H₂₇CO, C₁₅H₃₁CO or C₁₇H₃₅CO. Mixtures of such triacyl glycerides can also be used.

If present, triacyl glycerides are typically present in an amount of 5 to 30 wt.% with respect to the whole composition.

If additive(s) are present, they are typically present in an amount sufficient and not detrimental to the desired effect or effects to be achieved.

Generally, additive(s) may be present in the Initiator Paste or the Base Paste or in both parts, the Initiator Paste and the Base Paste.

If present, the additive(s) are typically present in the following amounts:
Lower amount: at least 0.005 or 0.01 or 0.1 wt.%;
Upper amount: at most 50 or 40 or 30 wt.%;
Range: 0.005 to 50 or 0.01 to 40 or 0.1 to 30 wt.%;
wt.% with respect to the weight of the whole composition.

The composition described in the present text typically comprises the respective components in the following amounts:
cationically curable pre-polymer: 5 to 80 wt.%,
initiator: 1 to 30 wt.%,
polyether-modified polydimethyl siloxane: 0.05 to 10 wt.%;
filler: 5 to 80 wt.%,
diluent: 2 to 80 wt.%,
additive(s): 0.005 to 50 wt.%,
wt.% with respect to the weight of the whole composition.

The composition described in the present text may also comprises the respective components in the following amounts:
cationically curable pre-polymer: 15 to 70 wt.%,
initiator: 3 to 10 wt.%,
polyether-modified polydimethyl siloxane: 0.1 to 5 wt.%;
filler: 10 to 70 wt.%,
diluent: 2.5 to 70 wt.%,
additive(s): 0.005 to 50 wt.%,
wt.% with respect to the weight of the whole composition.

The curable composition described in the present text may also be characterized by one or more of the following properties alone or in combination
a) viscosity of Base Paste and/or Initiator Paste before curing: 2 to 500 Pa*s or 30 to 150 Pa*s at 23°C;
b) consistency: 0, 1, 2 or 3 if determined according to ISO 4823:2015-08;
c) hardening within 15min within a temperature range of 20 to 40°C to a rubber elastic mass after mixing the Base Paste and the Initiator Paste;
d) the composition hardening within 15 min within a temperature range of 20 to 40°C to a rubber elastic mass after mixing the Base Paste and the Initiator Paste;
e) initial water-contact angle: equal to or smaller than 90° after mixing the Base Paste and the Initiator Paste.

A combination of the properties a) and c), a) and e), or a), c) and e) is sometimes preferred.

Described is also the cured composition obtained after hardening the curable composition described in the present text.

The cured composition can typically be characterized by one or more of the following features:
a) Shore hardness A: 40 to 90 or 50 to 90, if determined according to DIN 53505:2000-8 24h after mixing the Base Paste and the Initiator Paste;
b) tensile strength: at least 0.8 MPa or at least 1.0 MPa according to DIN 53504:2015-08;
c) elongation at break: at least 80%, if determined according to DIN 53504:2015-08, 24h after mixing the Base Paste and the Initiator Paste.
d) being rubber elastic.

A combination of the properties a) and b), a) and c), or a), b) and c) is sometimes preferred.

If desired, these parameters can be determined by means known to the skilled person or as described in the example section.

Further compositions are described below:
A composition provided as a kit of parts comprising a Base Paste and an Initiator Paste being separated before use,
the Base Paste comprising
   a cationically curable pre-polymer comprising a polyether backbone and at least two aziridino moieties and having a molecular weight of 150 to 20,000 g/mol, filler selected from reinforcing fillers, non-reinforcing fillers and mixtures thereof,
the Initiator Paste comprising
   initiator being selected from Lewis acids, Broensted acids or precursors of Lewis acids, Broensted acids, or mixtures thereof,
   filler selected from reinforcing fillers, non-reinforcing fillers and mixtures thereof, diluent,
the Base Paste or the Catalyst Paste or the Base Paste and the Catalyst Paste comprising in addition a polyether-modified polydimethyl siloxane of the following formula 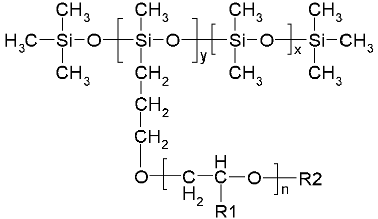 with
   R¹ being selected from hydrogen or methyl,
   R² being selected from H, C₁ to C₄ alkyl, or acetoxy,
   n = 1 to 50 or 1 to 30 or 1 to 20,
   x = 1 to 100, y = 1 to 20,
   the polyether surfactant having a solubility of at least 9.1 wt.% and a surface tension in the range of 24 to 30 dyn/cm.

A composition provided as a kit of parts comprising a Base Paste and an Initiator Paste being separated before use,
the Base Paste comprising
   a cationically curable pre-polymer comprising a polyether backbone and at least two aziridino moieties and having a molecular weight of 150 to 20,000 g/mol, filler selected from reinforcing fillers, non-reinforcing fillers and mixtures thereof,
the Initiator Paste comprising
   initiator being selected from Lewis acids, Broensted acids or precursors of Lewis acids, Broensted acids, or mixtures thereof,
   filler selected from reinforcing fillers, non-reinforcing fillers and mixtures thereof, diluent,
the Base Paste or the Catalyst Paste or the Base Paste and the Catalyst Paste comprising in addition a polyether-modified polydimethyl siloxane of the following formula 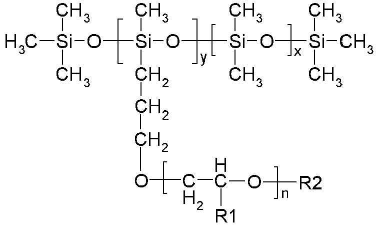 with
   R¹ being selected from hydrogen or methyl,
   R² being selected from H, C₁ to C₄ alkyl, or acetoxy,
   n = 1 to 50 or 1 to 30 or 1 to 20,
   x = 1 to 100, y = 1 to 20,
   the polyether surfactant having a solubility of at least 9.1 wt.% and a viscosity in the range of 100 cSt to 500 cSt and a surface tension in the range of 24 to 30 dyn/cm.

A composition provided as a kit of parts comprising a Base Paste and an Initiator Paste being separated before use,
the Base Paste comprising
   a cationically curable pre-polymer comprising a polyether backbone and at least two aziridino moieties and having a molecular weight of 150 to 20,000 g/mol, filler selected from reinforcing fillers, non-reinforcing fillers and mixtures thereof,
the Initiator Paste comprising
   initiator being selected from Lewis acids, Broensted acids or precursors of Lewis acids, Broensted acids, or mixtures thereof,
   filler selected from reinforcing fillers, non-reinforcing fillers and mixtures thereof, diluent,
the Base Paste or the Catalyst Paste or the Base Paste and the Catalyst Paste comprising in addition a polyether-modified polydimethyl siloxane of the following formula 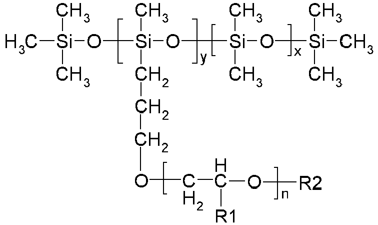 with
   R¹ being selected from hydrogen or methyl,
   R² being selected from H, C₁ to C₄ alkyl, or acetoxy,
   n = 1 to 50 or 1 to 30 or 1 to 20,
   x = 1 to 100, y = 1 to 20,
   the polyether surfactant having a solubility of at least 9.1 wt.%, a surface tension in the range of 24 to 30 dyn/cm and a viscosity in the range of 100 cSt to 500 cSt and being present in an amount of 0.1 to 5 wt.% with respect to the whole composition.

The composition described in the present text can be produced by mixing the respective components, e.g. by using a speed mixer, dissolver or a kneading machine.

The composition described in the present text is provided in separate parts before use. This is beneficial for improving the storage stability and/or shelf life.

When used, the individual parts or pastes are mixed in the suitable amounts and applied using conventional techniques.

Providing a Base Paste and an Initiator Paste with viscosities in the same range may facilitate the mixing to obtain a homogeneous composition, especially if the mixing is done using a static mixing tip.

The volume ratios of Base Paste to Initiator Paste can range of 10:1 to 1:10. Particularly preferred volume ratios of Base Paste to Initiator Paste are 1:1 to 10:1 or 2:1 to 5:1 (e.g. 5 parts of Base Paste to 1 part of Initiator Paste) or 2:1 to 4:1.

The curable composition described in the present text is typically stored in a container until use.

If the composition is applied into the sulcus of a teeth (i.e. the region between gum and hard dental tissue), using a container with a nozzle can be beneficial. Suitable containers with a nozzle are described in WO 2009/151983 A2 and are particular useful due to its specific geometry.

Cartridges which can also be used are described e.g. in US 2007/0090079 (Keller) or US 5,918,772 (Keller et al.). These kinds of cartridges are also commercially available e.g. from SulzerMixpac AG (Switzerland).

Other suitable devices can be found in WO 2005/016783 A1 (3M), WO 2007/047381 (3M), WO 2007/104037 (3M), and WO 2009/061884 (3M).

If desired, the composition can also be stored in foil bags.

A further improvement of the handling properties of dental compositions can be seen in using an automatic mixing and metering systems for two-component compositions which have automatic conveying and mixing units, such as are described e.g. in US 5,249,862, US 5,286,105 and US 5,419,460. The need for manual mixing of base pastes and catalyst pastes, above all when mixing larger quantities of material, can be eliminated, since this can take place automatically and within a short period of time. The result is usually a homogeneous product which is essentially free of air bubbles. Commercially available devices are distributed e.g. by 3M Oral Care under the brand Pentamix^{™} or Pentamix^{™} 2 or Pentamix^{™} 3.

In practice, the composition provided as a two-component system is typically syringed through a static or dynamic mixing device.

The composition can be applied onto a surface or into an impression tray or onto the patients' teeth or tissue (including sulcus) and placed in the patients' mouth. The composition may also be applied using an applicator like an elastomer syringe (application syringe).

Described is also a process of taking a dental impression, the process comprising the steps of
mixing the Base Paste and the Initiator Paste of the composition described in the present text to obtain a Mixed Paste,
applying the Mixed Paste to a surface, in particular to the surface of dental tissue,
letting the Mixed Paste harden,
removing the hardened composition from the surface.

The surface can be the surface of soft or hard oral tissue, the surface of an impression material, preferably of a cured impression material, the surface of a crown or the surface of a model of a tooth stump.

According to one embodiment, the composition described in the present text is not only suitable as dental impression material but also as dental retraction material.

The composition can not only be easily placed in the sulcus of a tooth, but also exerts sufficient pressure on the surrounding soft tissue having the result that the sulcus is widened. Due to its elastomeric properties in its cured stage, the composition can also be easily removed from the sulcus after hardening.

If used in the dental field, curing is preferably carried out at a temperature below 50 °C and preferably below 40 °C.

A typical time for cure of curable compositions as described in the present text used for dental applications is within 20 min, or preferably within 10 min, after mixing the components of the composition.

The material is generally regarded as cured, if the cured material fulfils the requirements for its use. For example, a dental precision impression material typically fulfils the requirements for its use when it fulfils the requirements of ISO 4823:2015-08 (such as compatibility with gypsum, strain in compression, recovery from deformation, detail reproduction, linear dimensional change).

Described is also a process for taking a dental impression including sub-gingival parts and/or conducting a dental retraction.

The dental material or composition can be used as impression material or for the production of crowns and/or bridges, including temporary or long-term crowns and bridges. In the latter case, the composition is used as a mould to be filled with the (temporary or long term) crown and/or bridge material, which is typically based on polymerizable (meth)acrylates or similar chemical reactants.

The curable composition is especially useful for producing dental materials like precision impression materials, bite registration materials, duplicating materials, modelling materials, situation impression materials.

The composition can be used e.g. for making impressions of soft and hard dental tissue. This can be achieved simply, e.g. filling the material into a dental tray and putting the tray into the mouth of a patient.

If used in the dental field, curing is preferably carried out at a temperature below 50°C and preferably below 40°C, and more preferably below 30°C. A typical time for cure of curable compositions of the invention used for dental impressioning is within 20 min, or preferably within 10 min, after mixing the components of the composition. For dental duplicating applications or dental modelling applications that take place in the professional dental laboratory, cure times of up to 45 min is generally acceptable. In other applications (e.g., sealing, moulding, coating, adhesively fixing), other cure times may be typical and higher cure temperatures may be acceptable. Nevertheless, setting times in the range of 30 min or 1 hour can still be useful.

The oral setting time is given by the manufacturer in the instructions for use. According to DIN EN ISO 4823:2015-08 the elastomeric property recovery from deformation of the vulcanized material have to reach values of ≥ 96.5 % within the recommended oral setting time. In addition, according to DIN EN ISO 4823:2015-08 the elastomeric property strain in compression of the vulcanized material has to come up to a value within the range of 0.8 to 20.0 % for Type 0 and Type 1 materials and in the range of 2.0 to 20.0 % for Type 2 and Type 3 materials, respectively within the recommended oral setting time.

Thus, if the composition is to be used as dental impression material, appropriate working times are in a range of 20 s to 7 min or 30 s to 6 min at room temperature (23 °C). For impression materials oral setting times should be as short as possible. Suitable oral setting times are ≤ 6 min or ≤ 5 min.

Described is also a kit of parts comprising the composition described in the present text and the following items alone or in combination: dental impression tray, application syringe, dental retraction material, temporary crown and bridge material.

Dental impression trays and/or application syringes are typically used for applying the composition described in the present text during a dental impressioning process.

Dental retraction materials are often used for widening the sulcus of a tooth or teeth shortly before the dental impression is taken.

Temporary crown and bridge materials are typically used after a dental impression process has been completed for producing a temporary crown or bridge for the prepared tooth which should be restored.

The invention is directed to the use of the polyether-modified polydimethyl siloxane described in the present text for reducing the stickiness of the composition obtained after hardening the Base Paste and the Catalyst Paste described in the present text.

It was found that polyether-modified polydimethyl siloxane with the described chemical formula and having a certain viscosity are suitable for achieving this property.

The following examples are given to illustrate the invention.

### Examples

Unless otherwise indicated, all parts and percentages are on a weight basis, all water is de-ionized water, and all molecular weights are weight average molecular weight. Moreover, unless otherwise indicated all experiments were conducted at ambient conditions (23°C; 1013 mbar).

### Methods

### Consistency

If desired, the consistency of the composition can be determined according to ISO 4823:2015-08 with the following changes. The weight load is applied to all specimens 45 seconds after the end of the mixing.

### Setting Time

The setting time of the compositions was determined by measuring the tan δ (delta) value of the mixed base and catalyst paste in dependence on the time at 23°C and 50% humidity by using a MCR 300 rheometer (plate/plate measurement system, 1mm gap) from Anton Paar company. The setting time "tE" and the working time "tA" were determined with the software supplied with the instrument using a curve analysis via tangent method. As known to the skilled person in the art, the tan δ value is the quotient of the plastic and elastomeric portion of the composition.

### Shore Hardness

If desired, the Shore Hardness A can be determined according to DIN 53505:2000-8 using a "Härteprüfgerät Zwick 3100/Prüfeinrichtung 7206" (Zwick GmbH &Co. Ulm) as the measuring device.

### Tensile Strength and Elongation at Break

If desired, tensile strength and elongation at break can be measured according to DIN 53504:2015-08 form S2. The specimens are measured 24 hours after cure. For determination of the values five independent measurements is performed. A "Universalprüfmaschine Zwick 1435" (Zwick GmbH & Co. Ulm) is used as the measuring device.

### Viscosity

If desired, the viscosity can be measured at 23 °C using a ThermoHaake Rotovisco 1 device with a plate/plate system (diameter 35 mm) and a slit of 0.1 mm. A sample volume of 175 µl dosed by a pipette is used for the measurement. Twenty viscosity values (Pas) are recorded in linear order during the measurement period of 30sec at a constant shear rate of 1361 1/s. The values between 15 and 28sec. are used to calculate a mean value of the viscosity.

### Water Contact Angle Measurement of Uncured Composition

For the preparation of the test sample the mixed paste was subjected to an object slide and flattened to obtain a homogenous 200µm thick layer. The object slide was placed on the table of a Drop Shape Analyse System DSA 30 (Krüss GmbH, Hamburg). At the time of 45 sec after start of mixing 5 µl of water were placed onto the surface of the specimen and a video (25 frames per second) recording was started to observe the droplet on the surface. The water contact angle was then calculated from the recorded video evaluating the first measurable contact angle of the droplet on the surface. The data (video sequences) were evaluated by the "circle fitting method", another standard method for data evaluation (see G. Kugel, T. Klettke, J. A. Goldberg, J. Benchimol, R. D. Perry, S. Sharma, J. Prosthod. 2007, 16, 84-92).

### Water Contact Angle Measurement of Cured Composition

For the preparation of a test sample the mixed paste was subjected to an object slide and flattened to obtain a homogenous 200µm thick layer. The object slide was placed on the table of a Drop Shape Analyse System DSA 30 (Krüss GmbH, Hamburg). At the time of 15 to 20 min after start of mixing 5 µl of water were placed onto the surface of the specimen and a video (25 frames per second) recording was started to observe the droplet on the surface. The water contact angle was then calculated from the recorded video evaluating the first measurable contact angle of the droplet on the surface. The data (video sequences) were evaluated by the "circle fitting method", another standard method for data evaluation (see G. Kugel, T. Klettke, J. A. Goldberg, J. Benchimol, R. D. Perry, S. Sharma, J. Prosthod. 2007, 16, 84-92).

### Stickiness after Setting Under Water

The stickiness can be determined by its haptic behaviour. For the preparation of the test sample the mixed paste is placed on an object slide and flattened to obtain a homogenous 200µm thick layer. At the time of 60 sec after start of mixing the object slide is placed in water tempered to 35(±1)°C and stays there for the duration of intraoral setting time, given by manufacturer's instructions for use. In case of the described examples (see below) the intraoral setting time is 120 sec. After removing the object slide out of the water the surface of the sample is dried with a soft stream of compressed air. With a clean dry fingertip, the surface of the sample is checked periodically. For every check a new untouched part of the surface must be used. Determined is the time (in min) from start of mixing until the first time when the stickiness disappeared.

### Solubility of Surfactant

Distilled water (10.0 g) and surfactant (1.0 g) are put in a transparent vessel of 25 mL in an environment of 23°C, the resultant liquid is stirred with a stirrer bar and the turbidity of the liquid is visually observed. When the liquid is clear, it is determined that the surfactant has a solubility of 9.1 wt.% or more. When the liquid is not clear, 10 g of distilled water is further added to the liquid, the resultant liquid is stirred with a stirrer bar and the turbidity of the liquid is visually observed again. When the liquid becomes clear, it is determined that the surfactant has a solubility of 4.8 to 9.1 wt.%. When the liquid does not become clear, it is determined that the surfactant has a solubility less than 4.8 wt.%.

### Surface Tension

The surface tension is given in dyn/cm (mN/m) and was determined in de-ionized water at 25°C for a concentration of 1 wt.% surfactant according to DIN EN 14370 (Nov. 2004) using the "Ringmethode".

### Materials

| Name | Description |
|---|---|
| Impregum^{™} Penta Soft Quick Base | Aziridino polyether impression material, base paste (commercially available; 3M Oral Care) |
| Impregum^{™} Penta Super Quick Catalyst | Polyether impression material, catalyst paste (commercially available; 3M Oral Care) |
| Struksilon^{™} 8026 | Surfactant (Schill & Seilacher) |
| Xiameter^{™} OFX-0193 | Surfactant (Dow Corning) |
| Silwet^{™} L 77 | Surfactant (Momentive) |
| Table 1 | |

### Manufacturing Process - Base Paste

For examples 2-4 Impregum^{™} Penta Soft Quick base paste and the respective surfactant were mixed by using a planetary mixer with vacuum capabilities (Speedmixer DAC 600.1 VAZ-P). Mixing time was 2 x 60 seconds with 1700 U/min under vacuum (< 10 mbar).

| Components | CE 1 | Ex 2 | Ex 3 | CE 4 |
|---|---|---|---|---|
| Impregum^{™} Penta Soft Quick Base | 100.0 | 99.0 | 99.0 | 99.0 |
| Struksilon^{™} 8026 | - | 1.0 | - | - |
| Xiameter^{™} OFX-0193 | - | - | 1.0 | |
| Silwet^{™} L77 | - | - | - | 1.0 |
| Table 2: Composition of Base Paste; weight in [g] | | | | |

### Manufacturing Process - Hardening Composition

The compositions of the examples were obtained by mixing the Base Paste and the Catalyst paste by hand, using a spatula for about 30sec. until the pastes are homogenously mixed. The mixing ratio of base:catalyst is 5:1 by volume or 1.00:0.24 by weight.

| | CE 1 | Ex 2 | Ex 3 | CE 4 |
|---|---|---|---|---|
| Viscosity of surfactant (cSt) | - | 360 | 266 | 18 |
| Solubility of surfactant in water | - | >9.1% | >9.1% | <4.8 % |
| Surface tension of surfactant (dyn/cm) | - | 26.7 | 26.7 | 20.5* |
| Start of setting (min) | 1.50 | 1.75 | 1.74 | 1.74 |
| End of setting (min) | 3.75 | 4.07 | 4.17 | 3.88 |
| Contact angle on unset paste, initial (°) | 95 | 69 | 65 | 93 |
| Stickiness after setting under water (min) | 8 | 7.5 | 9 | 11 |
| Table 3: Properties | | | | |

| | | | | |
|---|---|---|---|---|
| (* according to specification provided by the manufacturer for a concentration of 0.1 wt.%) | | | | |

Compositions containing a polyether-modified polydimethyl siloxane as described in the present text show not only acceptable curing properties but also an improved water contact angle on the unset paste compared to compositions containing a surfactant having a low water-solubility.

Further, compositions containing a polyether-modified polydimethyl siloxane as described in the present text are less sticky after setting under water compared to compositions containing a surfactant having a low water-solubility.

## Claims

1. Use of a polyether-modified polydimethyl siloxane for reducing the stickiness of a dental impression composition provided as a kit of parts comprising a Base Paste and an Initiator Paste being separated before use,
the Base Paste comprising
a cationically curable pre-polymer comprising a polyether backbone and at least two reactive functional moieties,
filler,
the Initiator Paste comprising
initiator being able to start the hardening of the polyether pre-polymer, filler,
diluent,
the Base Paste or the Catalyst Paste or the Base Paste and the Catalyst Paste comprising in addition a polyether-modified polydimethyl siloxane of the following formula 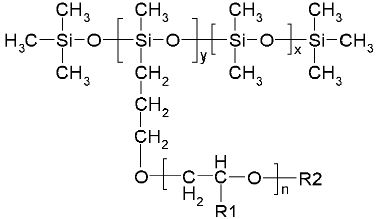 with
R¹ being selected from hydrogen or methyl,
R² being selected from H, C₁ to C₄ alkyl, or acetoxy,
n = 1 to 50, x = 1 to 100, y = 1 to 20,
the polyether-modified polydimethyl siloxane having a solubility in water of at least 9.1 wt.% and a surface tension in the range of 24 to 30 mN/m (dyn/cm), the solubility in water and the surface tension are determined as described in the description.

2. The use according to claim 1, the polyether-modified polydimethyl siloxane having a viscosity in the range of 100 to 500 mm²/s (cSt), the viscosity is determined as described in the description.

3. The use according to any of claims 1 to 2, the polyether-modified polydimethyl siloxane being present in an amount of 0.05 to 10 wt.% with respect to the whole composition.

4. The use according to any of claims 1 to 3, the polyether-modified polydimethyl siloxane being present in the Base Paste.

5. The use according to any of claims 1 to 4, the Base Paste and Initiator Paste being provided in a ratio of 1:1 to 10:1 with respect to volume.

6. The use according to any of claims 1 to 5, the dental impression composition comprising the components in the following amounts:
cationically curable pre-polymer: 5 to 80 wt.%,
initiator: 1 to 30 wt.%,
polyether-modified polydimethyl siloxane: 0.05 to 10 wt.%;
filler: 5 to 80 wt.%,
diluent: 2 to 80 wt.%,
wt.% with respect to the whole composition.

7. The use according to any of claims 1 to 6, the cationically curable pre-polymer comprising at least 2 aziridino groups and being **characterized by** the following features alone or in combination:
the aziridino groups having the following formula: 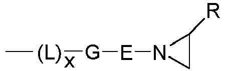 wherein
R represents H, C₁-C₁₂ alkyl, C₂-C₁₂ alkenyl, C₂-C₁₂ alkinyl, C₇-C₁₅ alkylaryl, C₇-C₁₅ arylalkyl or C₃-C₁₂ cycloalkyl, wherein hydrogen atoms can be replaced by Cl or F and/or wherein up to 5 carbon atoms can be replaced by atoms or group of atoms selected from O, CO, N or S,
E represents a C₁-C₁₈ branched or unbranched hydrocarbon chain wherein up to 5 carbon atoms can be replaced by atoms or group of atoms selected from O, CO, N or S,
G represents a group selected from C(O)O, C(O)NR, C(O), C(O)C(O), C(O)(CH₂)ₘC(O) with m = 1 to 10, C(S)NR or CH₂,
L represents O, S or NR, with x = 0 or 1,
molecular weight, Mn: 150 to 20,000 g/mol;
viscosity: 10 to 500 Pa*s at 23°C.

8. The use according to any of claims 1 to 7, the initiator being selected from Lewis acids, Broensted acids or precursors of Lewis acids, Broensted acids, or mixtures thereof.

9. The use according to any of claims 1 to 8, the initiator comprising at least one structural element of the following formula 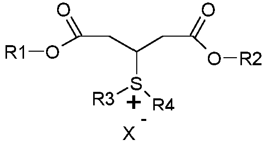 wherein
X⁻ is a non or low coordinating anion,
R1, R2, R3 and R4 are independently linear, cyclic or branched C₁ - C₂₀ alkyl or alkylene groups, wherein one or more of the methylene groups may be substituted by -CO-, -CONH-, -CON(CH₃)-, -S- and/or -O-, and wherein R1, R2, R3 and/or R4 can act as a bridging element connecting two or more structural elements.

10. The use according to any of claims 1 to 9, the dental impression composition being **characterized by** the following features alone or in combination:
consistency: 0, 1, 2 or 3 according to ISO 4823:2015-08;
viscosity of Base Paste and/or Initiator Paste: 2 to 500 Pa*s at 23°C;
Shore hardness A: 40 to 90, determined according to DIN 53505:2000-8 24h after mixing the Base Paste and the Initiator Paste;
tensile strength: at least 0.8 MPa according to DIN 53504:2015-08 after mixing the Base Paste and the Initiator Paste;
elongation at break: at least 80% according to DIN 53504:2015-08 after mixing the Base Paste and the Initiator Paste;
the composition hardening within 15 min within a temperature range of 20 to 40°C to a rubber elastic mass after mixing the Base Paste and the Initiator Paste;
initial water-contact angle: equal to or lower than 90° after mixing the Base Paste and the Initiator Paste.

11. The use according to any of claims 1 to 10, the dental impression composition comprising in addition additives selected from pigment(s), flavour(s), stabilizer(s), retarder(s), astringents(s), triacyl esters of glycerol, and mixtures thereof.

## Patentansprüche

1. Verwendung eines polyethermodifizierten Polydimethylsiloxans zur Verringerung der Klebrigkeit einer Zahnabdruckzusammensetzung, die als Teilesatz bereitgestellt wird, der eine Basispaste und eine Initiatorpaste umfasst, die vor der Verwendung getrennt sind,
die Basispaste aufweisend
ein kationisch härtbares Präpolymer, aufweisend ein Polyether-Grundgerüst und mindestens zwei reaktive funktionelle Einheiten, Füllstoff,
die Initiatorpaste aufweisend
Initiator, der in der Lage ist, die Härtung des Polyether- Präpolymers zu starten, Füllstoff, Verdünnungsmittel,
wobei die Basispaste oder die Katalysatorpaste oder die Basispaste und die Katalysatorpaste zusätzlich ein polyethermodifiziertes Polydimethylsiloxan der folgenden Formel aufweisen 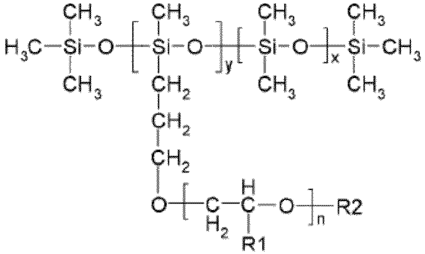 worin
R¹ ausgewählt ist aus Wasserstoff oder Methyl,
R² ausgewählt ist aus H, C₁- bis C₄-Alkyl oder Acetoxy,
n = 1 bis 50, x = 1 bis 100, y = 1 bis 20,
das polyethermodifizierte Polydimethylsiloxan eine Wasserlöslichkeit von mindestens 9,1 Gew.-% und eine Oberflächenspannung im Bereich von 24 bis 30 m N/m (dyn/cm) aufweist, wobei die Wasserlöslichkeit und die Oberflächenspannung wie in der Beschreibung angegeben bestimmt werden.

2. Die Verwendung nach Anspruch 1, wobei das polyethermodifizierte Polydimethylsiloxan eine Viskosität im Bereich von 100 bis 500 mm²/s (cSt) aufweist, die Viskosität wie in der Beschreibung beschrieben bestimmt wird.

3. Die Verwendung nach einem der Ansprüche 1 bis 2, wobei das polyethermodifizierte Polydimethylsiloxan in einer Menge von 0,05 bis 10 Gew.-%, bezogen auf die Gesamtzusammensetzung, vorhanden ist.

4. Die Verwendung nach einem der Ansprüche 1 bis 3, wobei das polyethermodifizierte Polydimethylsiloxan in der Basispaste vorhanden ist.

5. Die Verwendung nach einem der Ansprüche 1 bis 4, wobei die Basispaste und Initiatorpaste in einem Verhältnis von 1:1 bis 10:1, bezogen auf das Volumen, bereitgestellt sind.

6. Die Verwendung nach einem der Ansprüche 1 bis 5, die Zahnabdruckzusammensetzung aufweisend die Komponenten in den folgenden Mengen:
kationisch härtbares Präpolymer: 5 bis 80 Gew.-%,
Initiator: 1 bis 30 Gew.-%,
polyethermodifiziertes Polydimethylsiloxan: 0,05 bis 10 Gew.%,
Füllstoff: 5 bis 80 Gew.-%,
Verdünnungsmittel: 2 bis 80 Gew.-%,
Gew.-%, bezogen auf die gesamte Zusammensetzung.

7. Die Verwendung nach einem der Ansprüche 1 bis 6, wobei das kationisch härtbare Präpolymer mindestens 2 Aziridinogruppen aufweist und durch die folgenden Merkmale allein oder in Kombination **gekennzeichnet ist:**
die Aziridinogruppen weisen die folgende Formel auf: 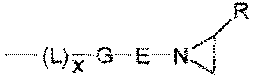 worin
R für H, C₁ -C₁₂-Alkyl, C₂-C₁₂-Alkenyl, C₂-C₁₂-Alkinyl, C₇-C₁₅-Alkylaryl, C₇ -C₁₅-Arylalkyl oder C₃-C₁₂-Cycloalkyl steht, wobei Wasserstoffatome durch C1 oder F ersetzt sein können und/oder wobei bis zu 5 Kohlenstoffatome durch Atome oder Atomgruppen ausgewählt aus O, CO, N oder S ersetzt sein können,
E für eine verzweigte oder unverzweigte C₁-C₁₈-Kohlenwasserstoffkette steht, wobei bis zu 5 Kohlenstoffatome durch Atome oder Atomgruppen ausgewählt aus O, CO, N oder S ersetzt sein können,
G für eine Gruppe steht ausgewählt aus C(O)O, C(O)NR, C(O), C(O)C(O), C(O)(CH₂)ₘC(O) mit m = 1 bis 10, C(S)NR oder CH₂,
L für O, S oder NR steht, wobei x = 0 oder 1, Molekulargewicht (Mn): 150 bis 20.000 g/mol;
Viskosität: 10 bis 500 Pa*s bei 23 °C.

8. Die Verwendung nach einem der Ansprüche 1 bis 7, wobei der Initiator ausgewählt ist aus Lewis-Säuren, Broensted-Säuren oder Vorläufern von Lewis-Säuren, Broensted-Säuren oder Mischungen davon.

9. Die Verwendung nach einem der Ansprüche 1 bis 8, wobei der Initiator mindestens ein Strukturelement der folgenden Formel umfasst 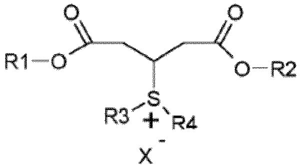 worin
X⁻ ein nicht oder schwach koordinierendes Anion ist,
R1, R2, R3 und R4 unabhängig voneinander lineare, zyklische oder verzweigte C₁ - C₂₀-Alkyl- oder Alkylengruppen sind, wobei eine oder mehrere der Methylengruppen durch -CO-, -CONH-, -CON(CH₃)-, -S- und/oder -O- substituiert sein können, und wobei R₁, R₂, R₃ und/oder R₄ als ein zwei oder mehrere Strukturelemente verbindendes Brückenelement wirken können.

10. Die Verwendung nach einem der Ansprüche 1 bis 9, wobei die Zahnabdruckzusammensetzung **gekennzeichnet ist durch** die folgenden Merkmale allein oder in Kombination:
Konsistenz: 0, 1, 2 oder 3 gemäß ISO 4823:2015-08;
Viskosität der Basispaste und/oder Initiatorpaste: 2 bis 500 Pa*s bei 23 °C;
Shore-Härte A: 40 bis 90, bestimmt nach DIN 53505:2000-8 24h nach dem Mischen der Basispaste und der Initiatorpaste;
Zugfestigkeit: mindestens 0,8 MPa gemäß DIN 53504:2015-08 nach dem Mischen der Basispaste und der Initiatorpaste;
Bruchdehnung: mindestens 80 % gemäß DIN 53504:2015-08 nach dem Mischen der Basispaste und der Initiatorpaste;
Aushärten der Zusammensetzung innerhalb von 15 min in einem Temperaturbereich von 20 bis 40 °C zu einer gummielastischen Masse nach dem Mischen der Basispaste und der Initiatorpaste;
anfänglicher Wasserkontaktwinkel: gleich oder kleiner als 90° nach dem Mischen der Basispaste und der Initiatorpaste.

11. Die Verwendung nach einem der Ansprüche 1 bis 10, wobei die Zahnabdruckzusammensetzung zusätzlich Additive aufweist, die aus Pigment(en), Geschmacksstoff(en), Stabilisator(en), Verzögerer(n), Adstringens bzw. Adstringenzien, Triacylestern von Glycerin und Mischungen davon ausgewählt sind.

## Revendications

1. Utilisation de polyéther modifié polydiméthylsiloxane par un polyéther pour réduire l'adhésivité d'une composition d'empreinte dentaire fournie sous forme d'un kit de pièces comprenant une pâte de base et une pâte d'initiateur séparées avant l'utilisation,
la pâte de base comprenant
un pré-polymère à durcissement cationique comprenant un squelette polyéther et au moins deux groupements fonctionnels réactifs, une charge,
la pâte de l'initiateur comprenant
l'initiateur pouvant amorcer le durcissement du pré-polymère polyéther, de la charge, du diluant,
de la pâte de base ou de la pâte catalytique ou la pâte de base et la pâte catalytique comprenant en outre un polydiméthylsiloxane modifié par un polyéther de la formule suivante 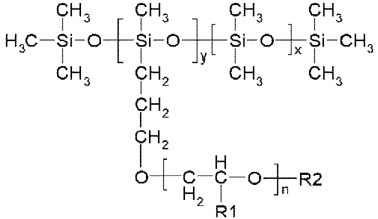 avec
R¹ étant choisi parmi l'hydrogène ou le méthyle,
R² étant choisi parmi H, l'alkyle en C₁ à C₄ ou l'acétoxy,
n = 1 à 50, x = 1 à 100, y = 1 à 20,
le polyéther modifié polydiméthylsiloxane ayant une solubilité dans l'eau d'au moins 9,1 % en poids et une tension superficielle comprise entre 24 et 30 mN/m (dyn/cm), la solubilité dans l'eau et la tension superficielle étant déterminées comme indiqué dans la description.

2. Utilisation selon la revendication 1, le polyéther modifié polydiméthylsiloxane ayant une viscosité comprise entre 100 et 500 mm²/s (cSt), la viscosité étant déterminée comme indiqué dans la description.

3. Utilisation selon l'une quelconque des revendications 1 à 2, le polyéther modifié polydiméthylsiloxane étant présent dans une plage allant de 0,05 à 10 % en poids par rapport à l'ensemble de la composition.

4. Utilisation selon l'une quelconque des revendications 1 à 3, le polyéther modifié polydiméthylsiloxane étant présent dans la pâte de base.

5. Utilisation selon l'une quelconque des revendications 1 à 4, la pâte de base et la pâte d'initiateur étant fournies dans un rapport de 1:1 à 10:1 par rapport au volume.

6. Utilisation selon l'une quelconque des revendications 1 à 5, la composition d'impression dentaire comprenant les composants dans les quantités suivantes :
le prépolymère à durcissement cationique : 5 à 80 % en poids,
l'initiateur : 1 à 30 % en poids,
le polyéther modifié polydiméthylsiloxane : 0,05 à 10 % en poids ;
le remplissage : 5 à 80 % en poids,
le diluant : 2 à 80 % en poids,
% en poids par rapport à la composition entière.

7. Utilisation selon l'une quelconque des revendications 1 à 6, le prépolymère à durcissement cationique comprenant au moins 2 groupes aziridino et étant **caractérisée par** les caractéristiques suivantes, seules ou en combinaison :
les groupes aziridino ayant la formule suivante : 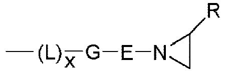 dans laquelle
R représente H, l'alkyle en C₁ à C₁₂, l'alcényle en C₂ à C₁₂, l'alcinyle en C₂ à C₁₂, l'alkylaryle en C₇ à C₁₅, l'arylalkyle en C₇ à C₁₅ ou le cycloalkyle en C₃ à C₁₂, dans laquelle les atomes d'hydrogène peuvent être remplacés par C1 ou F et/ou jusqu'à 5 atomes de carbone peuvent être remplacés par des atomes ou des groupes d'atomes choisis parmi O, CO, N ou S,
E représente une chaîne hydrocarbonée ramifiée ou non ramifiée en C₁ à C₁₈, dans laquelle jusqu'à 5 atomes de carbone peuvent être remplacés par des atomes ou des groupes d'atomes choisis parmi O, CO, N ou S,
G représente un groupe choisi parmi C(O)O, C(O)NR, C(O), C(O)C(O), C(O)(CH₂)ₘC(O) avec m = 1 à 10, C(S)NR ou CH₂,
L représente O, S ou NR, avec x = 0 ou 1, poids moléculaire (Mn): 150 à 20 000 g/mol ;
viscosité : 10 à 500 Pa·s à 23 °C.

8. Utilisation selon l'une quelconque des revendications 1 à 7, l'initiateur étant choisi parmi les acides de Lewis, les acides de Broensted ou les précurseurs d'acides de Lewis, les acides de Broensted, ou des mélanges de ceux-ci.

9. Utilisation selon l'une quelconque des revendications 1 à 8, l'initiateur comprenant au moins un élément structurel de la formule suivante 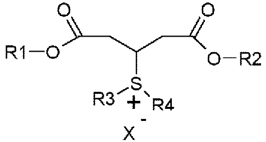 dans laquelle
X⁻ est un anion non ou faiblement coordinant,
R1, R2, R3 et R4 sont indépendamment des groupes alkyle ou alkylène en C₁ à C₂₀ linéaires, cycliques ou ramifiés, dans laquelle un ou plusieurs des groupes méthylène peuvent être substitués par -CO-, -CONH-, -CON(CH₃)-, -S-et/ou -O-, et dans laquelle R₁, R₂, R₃ et/ou R₄ peuvent servir d'élément de liaison entre deux ou plusieurs éléments structurels.

10. Utilisation selon l'une quelconque des revendications 1 à 9, la composition d'impression dentaire étant **caractérisée par** les caractéristiques suivantes, seules ou en combinaison :
la consistance : 0, 1, 2 ou 3 selon la norme ISO 4823:2015-08 ;
la viscosité de la pâte de base et/ou de la pâte initiatrice : 2 à 500 Pa·s à 23 °C ;
la dureté de shore A : 40 à 90, déterminée selon la norme DIN 53505:2000-8, 24 h après le mélange de la pâte de base et de la pâte initiatrice ;
la résistance à la traction : au moins 0,8 MPa selon la norme DIN 53504:2015-08 après mélange de la pâte de base et de la pâte d'amorce ;
l'allongement à la rupture : au moins 80 % selon la norme DIN 53504:2015-08 après mélange de la pâte de base et de la pâte d'amorce ;
la composition durcit en 15 minutes à une température comprise entre 20 et 40 °C pour devenir une masse élastique de caoutchouc après avoir mélangé la pâte de base et la pâte d'initiateur ;
l'angle de contact initial avec l'eau : égal ou inférieur à 90 ° après mélange de la pâte de base et de la pâte initiatrice.

11. Utilisation selon l'une quelconque des revendications 1 à 10, la composition d'empreinte dentaire comprenant en outre des additifs choisis parmi le(s) pigment(s), le(s) arôme(s), le(s) stabilisant(s), le(s) retardateur(s), le(s) astringent(s), les esters triacyliques de glycérol et les mélanges de ceux-ci.
